# EUROPEAN PATENT APPLICATION

(11) **EP 0 668 356 A2**
(43) Date of publication of application: **23.08.1995**
(21) Application number: 95870007.2
(22) Date of filing: 31.01.1995
(51) Int. Cl.: C12N 15/38, C12N 7/01, A61K 39/265, A61K 39/295, G01N 33/15

(54) **Recombinant infectious bovine rhinotracheitis virus mutant, methods for the production of same, and vaccines containing the same**

(30) Priority: 31.01.1994 ES 9400172
(71) Applicant: LABORATORIOS HIPRA, S.A., E-17170 Amer (Girona) (ES)
(72) Inventor: Rebordosa, Xavier, E-08221 Tarrasa (Barcelona) (ES); Pinol, Jaume, E-08190 Valldoreix(Sant Cugat del Valle) (ES); Perez-Pons, Josep Antoni, E-08017 Barcelona (ES); Lloberas, Jorge, E-08208 Barcelona (ES); Querol, Enrique, E-08021 Barcelona (ES)
(74) Representative: Claeys, Pierre

(57) **Abstract**

Infectious Bovine Rhinotracheitis Virus mutant, methods for the production of same, and vaccines containing the same. The obtained virus, characterized as BHV-1 FM gll⁻, results from one deletion, from one insertion, or from one substitution (deletion and insertion) in the gene gll (gE) from Bovine Herpesvirus type 1 (BHV-1), which fails to produce any antigenic polypeptide of glycoprotein gll (gE). BHV-1 FM gll⁻ virus is utilized in the preparation of vaccines for Infectious Bovine Rhinotracheitis. These vaccines allow to distinguish serologically between animals vaccinated with the same and animals infected with field strains of lBR virus.

## Description

### FIELD OF INVENTION

The present invention relates in general to viral vaccines based on mutant viruses and, specifically to the Infectious Bovine Rhinotracheitis Virus (Bovine Herpesvirus type 1), to the mapping, cloning and sequencing of the glycoprotein gII, and the generation of mutants containing deletions or insertions or both in the locus of the glycoprotein gII gene, and its utilization in a vaccine in the form of a defective virus for this glycoprotein, such that no antigenic polypeptide encoded by the viral gene are produced. Animals vaccinated with such mutant viruses do not develop antibodies against the viral glycoprotein gII and can be distinguished serologically from animals infected with field strains of Infectious Bovine Rhinothracheitis virus. The present invention also relates to vaccines for Infectious Bovine Rhinothracheitis containing the same, methods for production, formulation and utilization of the same and provide tools to distinguish serologically animals vaccinated with the same from animals infected with field strains of Infectious Bovine Rhinotracheitis virus.

### DEFINITION OF TERMS TO HELP THE INTERPRETATION OF THE INVENTION

The following definitions are provided for a clear and consistent understanding of the specifications stated in the claims.
**Adjacent:** Any position in the nucleotide sequence located immediately 5' or 3' to a defined sequence.
**Cell culture:** A proliferating mass of cells which may be in an undifferentiated or differentiated state. As used herein "cell" and "cell line" are used interchangeably. All such designations include progeny, but not all of the components of the progeny have an identical DNA sequence.
**Coding sequence:** A deoxyribonucleotide sequence which when transcribed and translated results in the formation of a cellular protein, or a ribonucleotide sequence which when translated results in the formation of a cellular protein.
**Control sequence:** Refers to DNA sequences necessary for the expression of an operative linked coding sequence in a particular host organism. The control sequences include promoter, enhancers, terminator signals, polyadenylation signals, and possibly, other still poorly understood sequences.
**Deletion:** Removal of a fragment from a DNA sequence, being the regions on either side joined together.
**Expression system:** Refers to DNA sequences containing a specific coding sequence and control sequences operably linked, so that hosts transformed with these sequences are able to produce the encoding protein. The expression system may be included on a vector, although sometimes, the relevant DNA may integrate into a chromosome of the host cell.
**Flanking sequence:** Refers to DNA sequences adjacent, in 5' and 3', to the coding sequence. They include the control sequences.
**Gene:** A discrete nucleic acid region which is responsible for a discrete cellular product (RNA and/or polypeptide).
**Glycoprotein:** Protein that contain covalently bonded sugar molecules.
**gII:** Glycoprotein II and gene of the glycoprotein gII from Bovine Herpesvirus type 1 (also named gE since the 18th International Herpesvirus Workshop held in Pittsburgh, Pennsylvania, in 1993).
**Insertion:** Introduction of an additional stretch of DNA sequence into another DNA sequence.
**Operative linked:** Refers to the functional association of the components. Thus, a coding sequence operatively linked to control sequences refers to an association wherein the coding sequence can be expressed under the control of these sequences.
**p.f.u.:** plaque forming units.
**pb:** a complementary pair of bases of the double strand DNA. **kb** refers to 1000 bp.
**Reading frame:** Segment of DNA encoding a polypeptide or polypeptides.
**Sequence homology:** Indicates different nucleotide sequences that are functionally equivalent to one another. The nucleotide differences do not affect the functionality of the products encoded in the different sequences.
**Substitution:** Replacement of a stretch of DNA sequence by another DNA sequence (corresponds to a deletion plus an insertion).
**Transformants:** Refers to cells that, through different mechanisms, acquire foreign DNA sequences, which can or cannot induce changes in the host cells.
**Vector:** DNA sequence suitable to carry other DNA sequences.
**+/-:** Refers to the presence/absence of a functionality in a cell or virus.

### 1.- BACKGROUND OF THE INVENTION.

### Infectious Bovine Rhinotracheitis

Bovine Herpesvirus type 1 (hereinafter "UBHV-1"), also known as Infectious Bovine Rhinotracheitis virus (hereinafter "IBRV") is an agent associated with a wide spectrum of pathologies such as respiratory problems (the main cause of the loss of growth and vitality in calves), reproductive problems (abortions, infertility and sterility), losses in the milk yield, enteritis, meningitis, conjunctivitis, problems in the central nervous system and dermal infections of cattle (Timoney, J.F. et al. (1988) in Hagan and Bruner's Microbiology and Infectious Diseases of Domestic Animals Comstock Publishing Associates, 8th ed., Ithaca. pp 594-601). The severity of the illness resulting from IBRV infections depends on the virus strain and on the age of the affected animal . From the above problems, respiratory problems are the most common. The estimated incidence of the respiratory problems is 2% in adults and 10-30% in calves, being the average of calf mortality about 10%. In calves, the annual worldwide losses are estimated in 3000 million $.(Animal Farm, Supplement 17, May 1992).

As a member of the *Alphaherpesviridae* family, BHV-1 is highly neurotropic, remaining latent in the nervous system of the animal after recovering from infection. The animals may present signs of recurrent disease without being reexposed to the virus (Rock, D.L. et al. (1986) J. Gen. Virol., 67: 2515-2520). This fact is extremely important for artificial insemination programs since infected bulls produce recurrent shedding of viruses. Some pharmacological treatments such as dexamethasone can also provoke nasal shedding of the virus with or without clinical symptoms of active Infectious Bovine Rhinothracheitis (hereinafter "IBR"). As a consequence, new outbreaks can result from viruses latent in infected but asymptomatic cattle. Thus, the design of vaccines and differential diagnostic systems, specially for eradication programs, could be very useful.

### BHV-1 genome and proteins

The genome of BHV-1 consists of a linear, double-stranded, DNA molecule, approximately 135 kb in size. This genome is arranged in two unique sequences, the long one of about 100 kb (named UI) and the short one of about 13 kb (named Us). The Us is bracketed by two inverted repeat sequences: the internal (hereinafeter "Internal Repeat short" or "IRs") and the terminal (hereinafter "Terminal Repeat short" or "TRs"), both of about 11.5 kb (Hammerschmidt, W. et al. (1986) J. Virol., 58:43-49; Mayfield, J.E. et al. (1983) J. Virol., 47: 259-264). This genome structure corresponds to the herpesvirus class D which is also found in the pseudorabies virus (hereinafter "PRV"), equine herpesvirus types 1 and 3 (hereinafter "EHV-1, -3"), and varicellazoster virus (hereinafter "VZV"). A characteristic of the herpesvirus genome is the ability to invert the orientation of unique sequences flanked by inverted repeated sequences (Us in the above viruses) leading two equimolar isoforms of the BHV-1 genome.

Restriction maps of the genomes from several IBRV strains have been reported (Mayfield, J.E. et al. (1983) J. Virol., 47: 259-264; Simard C. et al. (1990) Arch. Virol., 110: 63-75 ) and three different groups of viral genomes have been established, without any correlation with the epidemiology (Engels, M. et al. (1986) Virus Res., 6:57-73).

Although BHV-1 genome may encode more than 33 structural polypeptides (Misra, V. et al. (1981)) J. Virol., 40:367-378), few of them have been identified and mapped in the IBRV genome.

### BHV-1 glycoproteins

From the encoded structural polypeptides, 11 out of 33 are glycosylated (Misra, V. et al. (1981) J. Virol., 40: 367-378; Van Drunen Littel-van den Hurk, S. et al. (1986) J. Virol., 59: 401-410; Badia & Querol (1988) J. Virol. Meth., 22: 23-29). Glycoproteins play an outstanding role in the recognition, binding and penetration of the virus into the host cell, as well as in the immune response of the infected animal (virus neutralization, destruction of the infected cell and induction of the mechanisms of cell immunity). In addition, sugars play an important role in the generation of the functional glycoprotein, and also in the recognition and immunological properties of such glycoproteins (Olofsson S. (1992) APMIS Suppl 267, 100: 84-95; Van Drunen Littel-van den Hurk, S., et al. (1990) J. Gen. Virol., 71: 2053-2063).

Monoclonal antibodies raised against BHV-1 have led to the identification and characterization of the four main envelope glycoproteins, gI, gII, gIII and gIV. The gI glycoprotein is synthesized as a 105 kDa (relative molecular mass) polypeptide, which in the mature form (fully glycosylated and proteolytically processed) yields a heterodimer of 130 kDa (built from two disulfide-bonded monomers of 75 and 55 kDa respectively). The precursor of gII glycoprotein is a nonglycosylated 90 kDa polypeptide, 108 kDa when glycosylated. The gIII glycoprotein is isolated in the form of a 180 kDa homodimer from a mature monomer (N- and O-glycosylated) with relative molecular mass of 91 kDa. The glV glycoprotein has a relative molecular mass of 71 kDa in a mature form (N- and O-glycosylated), and is also present as a 140 kDa homodimer. These proteins can also be found in the surface of BHV-1 infected cells, and react with neutralizing monoclonal antibodies or monospecific antiserum (Van Drunen Littel-van den Hurk, S. et al. (1984) Virology. 135: 466-479; Van Drunen Littel-van den Hurk, S. et al. (1986) J. Virol., 59: 401-410; Marshall, R.L. et al., (1986) J. Virol., 57: 745-753; Fitzpatrick, DR., et al. (1988) J. Virol., 62: 4239-4248; Van Drunen Littel-van den Hurk, S. et al. (1989) J. Virol. 63: 2159-2168).

Several herpesvirus proteins are involved both in the formation of virus-neutralizing antibodies and in lymphocyte-mediated cytotoxic response (Norrild, B. et al. (1979) J. Virol., 32: 741-748; Balachandran, N. et al. (1982) Infect. Immun., 37: 1132-1137; Hampl, H. et al. (1984) J. Virol., 52: 583-590; Ben Porat, T. et al. (1986) Virology, 154: 325-334; and Wathen, L.M.K. et al. (1985) Virus Res., 4: 19-29). It is also described that BHV-1 gI, gIII and gIV are the main glycoproteins involved in eliciting neutralizing antibodies (Babiuk, L.A. et al. (1987) Virology, 159: 57-66). These results are important to design a protein defective viral strain that can be used as a vaccine. A defective vaccine allows to distinguish between vaccinated animals and animals infected with field strains of BHV-1, acting the deleted protein as a marker to detect vaccinated animals. The protein to be deleted must be carefully selected. Such protein must not be essential for virus replicacation and, when possible, not involved in eliciting neutralizing antibodies. A good choice would be the deletion of a protein non essential for viral replication, but acting as a counter-measure against the host immune response. IBRV gl and glV glycoproteins are not useful for designing a defective vaccine because their homologous from HSV and PRV (gB, gD, gX and gp50) are either essential proteins for viral replication in cell cultures or play a significant role in the entry of the virus into the cells (Desai, W. et al. (1988) J. Virol., 62: 2596-2604; Ligas, W.L. and Johnson, D.C. (1988) J. Virol., 62: 1486-1494; Liang, X. (1991) J. Virol., 65: 1124-1132; Card, J.P. et al. (1992) J. Virol., 66: 3032-3041). It has been reported the existence of genes encoding glycoproteins dispensable for viral amplification in cell culture in the US region of several members of the Alphaherpesviridae family (for HSV-1, see Longnecker, R. et al. (1987) Science, 236: 573-576; for PRV see Petrovskis, E.A. et al. (1986) J. Virol., 60: 1166-1169; Petrovskis, E.A. et al. (1987) Virol., 159: 193-195; Mettenleiter, T.C. et al. (1987) J. Virol., 61: 2764-2769; and, Thomsen, D.R. et al. (1987) J. Virol., 61: 229-232). The gene encoding the BHV-1 gII glycoprotein would be homologous to the PRV gl glycoprotein gene, which has already led to a defective pseudorabies strain vaccine (Lomniczi, B. et al. (1984) J. Virol., 49: 970-979; Mettenleiter, T.C. et al. (1985) J. Virol., 53: 52-57). In the BHV-1 UI region is located a gene encoding the gIII glycoprotein which, although non essential for viral replication in cell culture, its deletion involves a lesser cell adsorption and a delayed viral replication (Liang, X. et al. (1991) J. Virol., 65:1124-1132).

Many viruses, among them the herpesvirus, present proteins that interfere with the host immune system- with cytokynes, with the complement system, etc.- (Gooding L. (1992) Cell, 71; 5-7). It has been reported for HSV that glycoproteins gE and gI bind to the IgG Fc domain, thus interfering with the complement mediated lysis of infected cells (Bell S. et al. (1990) J. Virol., 64; 2181-2186). These protein activities are dispensable for viral replication in cell culture as they act as a counter-measure against the host immune system (Gooding, L. (1992) Cell, 71; 5-7; Post L. and Roizman B. (1981) Cell, 25; 227-232). They represent the best choice for defective vaccine design because their absence does not prevent viral growth in cell culture and impede the interference with the host immune system.

The deletion of the PRV gI glycoprotein gene, homolugous to gII from BHV-1, alters the neurotropism and reduces the neurovirulence of PRV when injected intraocularly in rats (Card et al., (1992) J. Virol., 66, 3032-3041). This fact could enhance the biosecurity of a vaccine with a deletion in the gII glycoproptein. Considering overall data, the deletion of gII glycoprotein from BHV-1 presents additional advantages versus the deletion of other glycoproteins such as gIII: (a) as above mentioned, its homologous from HSV (gE glycoprotein) represents a counter-measure against the host immune system, and (b) an IBRV gII⁻ strain will present lesser neurovirulence. Other characteristics are shared also with gIII glycoprotein: (a) to be a viable defective viral strain, and (b) to be a protein immunogenic enough to allow the design of a kit to serologically distinguish animals vaccinated with these mutants from animals infected with field strains. For the above reasons, the gII glycoprotein from BHV-1, which is homologous to PRV gI and HSV gE proteins, is the target of the present invention.

To map and identify the glycoprotein gII (or gE) gene from BHV-1, it was assumed that gII gene, as in other herpesviruses, would be located in the Us region of the genome (Wirth U. et al. (1989) J. Virol., 65: 4882-4889). All reported data from other *Alphaherpesviridae* family members indicate the existence of a sequence containing 5 genes laying in conserved positions along the Us region of the genome. They are US4(gG), US5, US6(gD), US7(gI) and US8(gE) for HSV-1; US4(gG2), US5, US6(gD2), US7(gI2) and US8(gE2) for HSV-2; and gX, gp50, gp63 and gI for PRV (McGeoch D.J. (1990) J. Gen. Virol., 71: 2361-2367). VZV virus presents only the genes corresponding to US7 (gpIV) and US8 (gpI) because its Us is shorter than in the other herpesviruses. In addition, it has been recently reported the whole sequence of the EHV-1 genome and it corroborates the structure described for HSV-1 (Telford E. et al. (1992) Virology, 189:304-316).

In the present invention it has been identified, mapped in the Us region, cloned and sequenced the gene coding for the gII (gE-like) glycoprotein from BHV-1 or IBRV, and has been constructed a gII⁻ defective BHV-1 strain. In addition, it has been demonstrated that the gII gene is not essential for viral replication in cell cultures. Moreover, it has been demonstrated that gII glycoprotein is immunogenic, thus it combines those characteristics required for its use as a marker in the design of a vaccine able to further distinguish serologically between animals vaccinated and animals infected with field strains.

### Reported fundamental IBR vaccines

There exist several vaccines against IBR based on chemical or physically attenuated or killed virus. Laboratorios HIPRA S.A. has some of these vaccines. There are also vaccines obtained by attenuation of viruses by serial passages in cell cultures, alteration of the host cell or modification of culture conditions. There are also subunit vaccines prepared by solubilizing components from the viral envelope (i.e., by means of detergents) (Babiuk, L.A. et al. (1987) Virology, 159:57-66).

All of the above vaccines present disadvantages. Those using whole IBR virus, even killed, can induce allergic or hypersensitivity reactions. Subunit vaccines have a lesser toxicity and are suitable for the serologic differentiation between infected and vaccinated animals, although are expensive in terms of production and must be administered in several doses.

Vaccines based in modified-live viruses are more effective because they stimulate both the humoral and the cellular components of the immune system. Nevertheless, they do not eliminate latency. There have been reported several cases of reversion to virulent form upon utilization of modified-lived viruses. Moreover, there have been reported hazards of vaccine-induced abortions when administered intramuscularly. Intranasal inoculated vaccines seems to be more safety, but they present administration problems.

There have been reported vaccines designed from modified-live viruses, thymidine kinase negative (tk⁻) and temperature resistant mutant viruses (for example US patent applications numbers 4.569.840 and 4.703.011). However, these mutants do not solve the problem of the distinction between animals vaccinated with the mutant and animals infected with field strains. A vaccine which overcomes the problem of latency and allows the distinction between animals vaccinated with the mutants and animals infected with field strains is the EP application 88118266.1, which describes the design of a gIII-thymidine kinase double defective strain (gIII⁻tk⁻).

During the development of the present invention, there have been disclosed three applications for patents which deal on gE (or gII) glycoprotein from BHV-1. They correspond to applications PCT/US92/06034, PCT/FR93/0064 and PCT/NL92/00097. Application PCT/US92/06034 reports and claims the gE glycoprotein gene, its polypeptidic product and additional uses of them. However, the gene sequence has been defined incorrectly. They describe the encoded polypeptide as a sequence of 2038 bp, yielding a protein of 617 amino acid residues. This is not correct since there is a mistake derived from the erroneous choice of the initiation codon, which leads to an extended polypeptide and a promoter lacking the TATA box. This is supported by multialignment analysis of different gE glycoprotein sequences from several herpesvirus (Rebordosa et al., manuscript in preparation). Moreover, the putative 617 aa sequence would lose the N-terminal signal peptide (von Heijne G. (1986) Nucleic Acids Res., 14:4683-4690; and, Perlman D. and Halvorson H. O. (1983) J. Mol. Biol., 167:391-409). Therefore, it is almost impossible that a protein sequence such as the claimed by application PCT/US92/06034 could ever be expressed as a conformationally stable functional protein. Application PCT/FR93/0064 reports and claims a patent for the nucleotide sequence of a large portion from the BHV-1 Us region. Moreover, it claims for putative applications derived from the sequence but, to support their claims, they do not present experimental work other than the sequence. A third application is PCT/NL92/00097 which describes the design of a double defective strain (gE⁻tk⁻) by recombinant procedures. Nevertheless, the procedure described for deleting the gE gene is somewhat different to the described in the present application. Thus, application PCT/NL92/00097 reports a deletion involving the promoter region of the gE gene. This cannot allow the expression of a sequence inserted into the gE locus as they claim in claim 12 (at least under the control of gE promoter). The deletion of gE promoter may also interfere with genes downstream the gE gene (the US9 gene, a 10 kDa protein which may be essential for viral replication). In addition, gE and US9 genes share the termination sequence. In conclusion, the defective strain described in the present invention has advantages respect to that described in application PCT/NL92/00097, since a large portion of gE coding sequence has been deleted but maintaining both, the promoter and a short sequence encoding the signal peptide. Such a deletion yields a nonfunctional peptide and does not preclude the expression of downstream genes. Moreover, the gene construct described in the present application allows the insertion of genes to be expressed under the control of BHV-1 gE promoter. Finally, application PCT/NL92/00097 claims the expression of BHV-1 gE gene into a prokaryotic host. Presented data indicate that such expression is at very low levels and thus probably inadequate to detect BHV-1 in biological samples.

### 2.- SUMMARY OF THE INVENTION

An object of the present invention is to obtain a BHV-1 mutant virus characterized as BHV-1 FM gII⁻.

Another object of the present invention is to obtain a BHV-1 mutant virus, as a result of a deletion, insertion or substitution (deletion and insertion) in the gII gene from BHV-1.

Still another object of the invention is to obtain a BHV-1 mutant virus which fails to produce any antigenic gII polypeptide from gII glycoprotein as a result of deletion, insertion or both in the gII gene.

A further object of the present invention is to provide methods for the production of the mutant virus BHV-1 FM gII⁻.

Another object of the present invention are the vaccines effective in controlling the IBR which contain the BHV-1 FM gII⁻ virus and the methods of preparation.

The invention provides tools for further application in diagnostic methods to serologically distinguish animals vaccinated with BHV-1 FM gII⁻ mutant virus from animals infected with field strains.

### BRIEF DESCRIPTION OF THE DRAWINGS

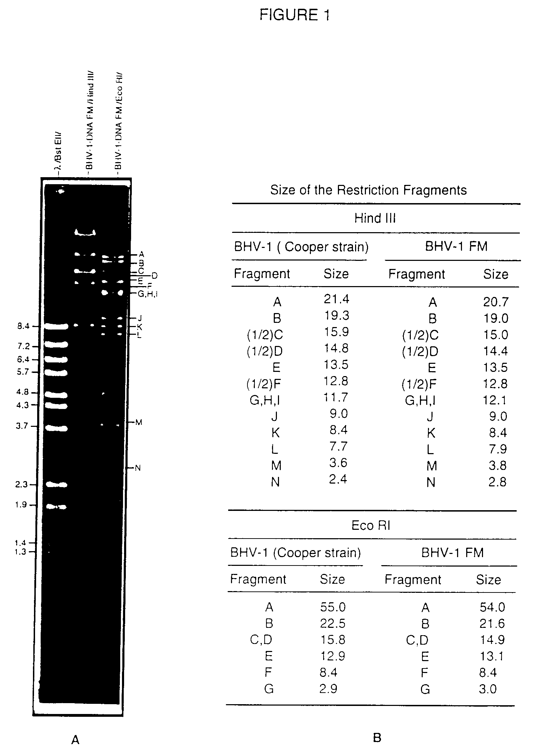

- **Figure 1A** shows the restriction enzyme analysis of the BHV-1 FM strain. The relative electrophoretic mobilities of the markers are in kb. On the right lane are the fragments from a Hind III digestion. The resulting molecular masses are compared to those reported by Mayfield et al. (1983) J.Virol., 47: 259-264, for the Cooper strain of BHV-1.1. The table (**Figure 1B**), shows the molecular masses (in kb) of the fragments from both strains. Both strains show the same Hind III pattern and a very similar EcoRI pattern. Therefore, BHV-1 FM strain belongs to the 1.1. subtype from Bovine herpesvirus.

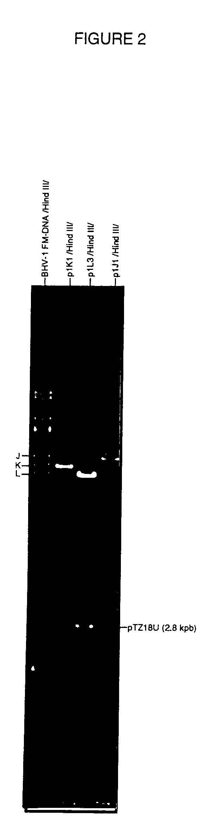

- **Figure 2** shows the HindIII digestion of the hybrid plasmids p1K1, p1L3 and p1J1 compared with a digestion with the same enzyme of the BHV-1 FM genome. The molecular mass of the fragment cloned into the recombinant plasmid p1K1 corresponds to the K-band from the Hind III digestion of BHV-1 FM DNA.

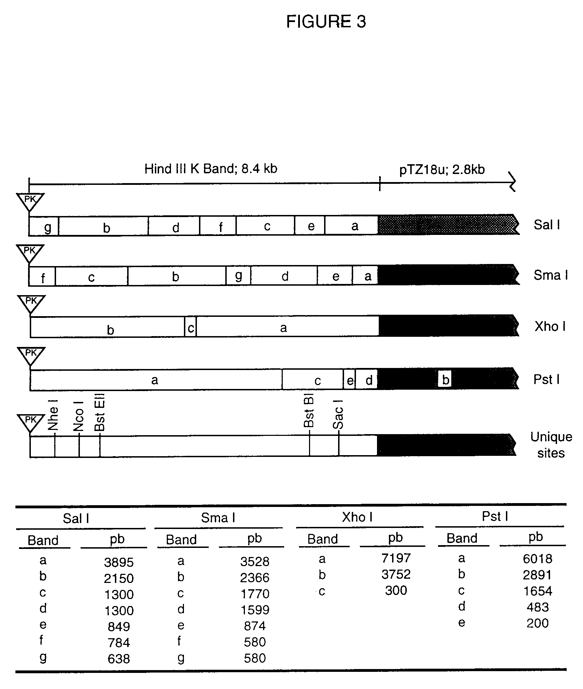

- **Figure 3** presents the scheme of the restriction map from the sequence cloned into the recombinant plasmid p1K1. Unique restriction sites (Nco I, BstE II, NheI , Sac I and Bst BI) and multiple restriction sites (Sma I, Sal I, Pst I and Xho I) in the cloned fragment are also indicated. The size in bp of the resulting fragments are shown in the table of this figure.

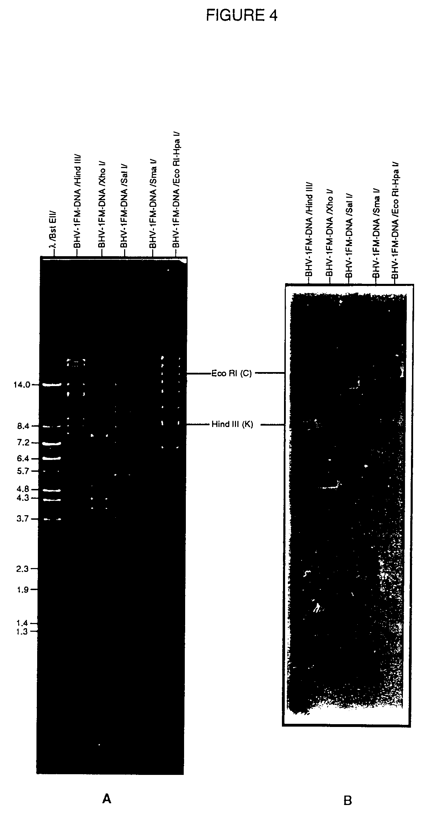

- **Figure 4A** shows the electrophoresis of BHV-1 FM DNA digested with Hind III, Xho I, Sal I, Sma I and Eco RI+Hpa I.
- **Figure 4B** shows the hybridization of fragments from digestion as indicated in Figure 4A with the SaI1a fragment described in Figure 3. The analysis of bands detected by the fragment Sal IA shows the existence of a Sal I-Eco RI (SE1) fragment of 6.2 kb which overlaps with the Hind III K fragment. This fragment was used to determine the 3' sequence of the glycoprotein gII gene and to construct the recombinant plasmid pΔgII.

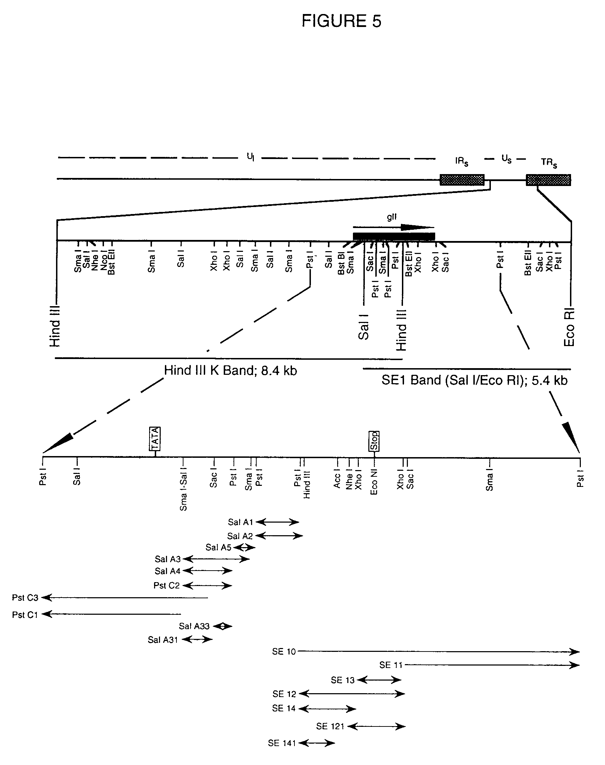

- **Figure 5** depicts a scheme of the regions of recombinant plasmids p1K1 and pSE1 used to sequence the gII glycoprotein gene from BHV-1 FM. The subclones and sequencing reactions are also indicated.

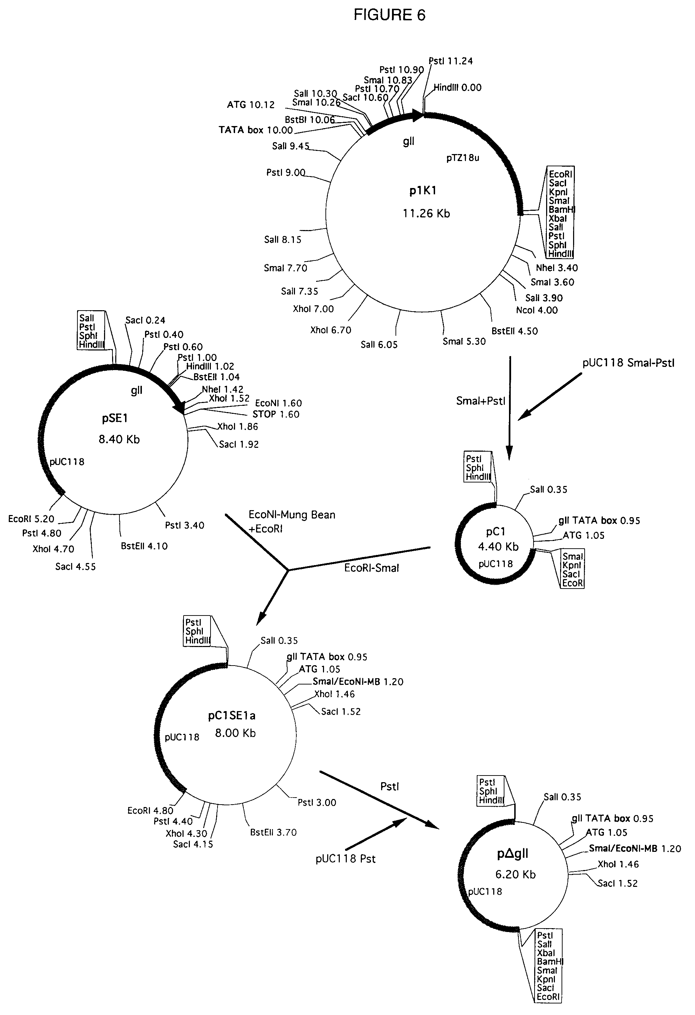

- **Figure 6** indicates the constructs designed in the present invention. Specifically, it shows the construction of the pΔgII plasmid cotransfected with DNA from BHV-1 FM gII⁺ to obtain the recombinant virus BHV-1 FM gII⁻. First, the recombinant plasmid p1K1 (described in Figure 2) was digested with restriction enzymes Sma I and Pst I. The 1.2 kb C1 fragment was isolated and subcloned into a pUC118 plasmid previously restricted with the same enzymes. The resulting recombinant plasmid was named pC1. In a similar way, the plasmid pSE1 (obtained by insertion of the Sal I-Eco RI 6.2 kb fragment from BHV-1 FM into a pUC118 vector) was restricted with the enzyme Eco NI and blunt ended with Mung Bean nuclease to eliminate the protruding 5' single stranded end: 5'-CCTCC3' 3'-GGAGGC5' yielding the following blunt end: 5'-CCTCC3' 3'-GGAGG5' Upon digesting the resulting linearized plasmid with enzyme Eco RI it was further cloned into the plasmid pC1 previously restricted with Sma I and Eco RI, yielding the recombinant plasmid pC1SE1a. Finally, the recombinant plasmid pC1SE1a was digested with the restriction enzyme Pst I and the resulting 3 kb fragment isolated and cloned into a pUC118 vector previously restricted with Pst 1. The resulting recombinant plasmid was named pΔgII.

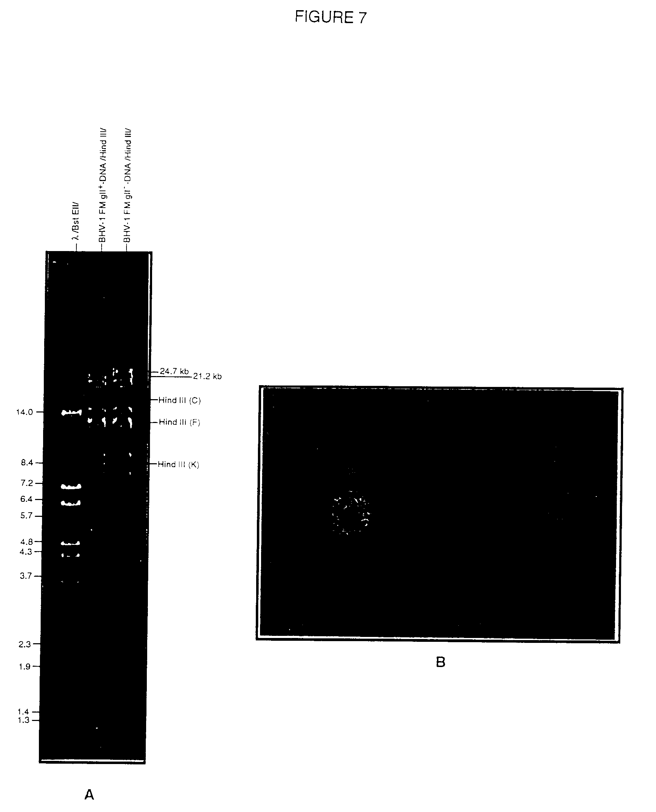

- **Figure 7A** shows the restriction pattern of BHV-1 FM gII⁻ DNA compared to that of BHV-1 FM gII⁺, upon digestion of both with the enzyme Hind III. **Figure 7B** indicates the procedure used to isolate recombinant gII⁻ viruses. Specifically, the figure shows the appearance of GBK cells after being cotransfected with infective BHV-1 FM gII⁺ and pΔgII DNA. After *in situ* hybridization (see Section **4**.**L**.) there appear dark plaques (Figure 7B(1)) and light plaques (Figure 7B(2)) with cytopathic effect. The probe utilized (see Section **4.L.**) detects plaques resulting from the infection by BHV-1 FM gII⁺, therefore light plaques should correspond to gII⁻ viral infection. Viral gII⁻ progeny was isolated (see Section **4.L.**) and its DNA analyzed by Hind III digestion. Figure 7A shows an agarose electrophoresis (stained with ethidium bromide) comparing the Hind III digestion of the starting strain BHV-1 FM gII⁺ and the mutant strain BHV-1 FM gII⁻. The mutant strain lacks bands C, F y K, and presents two additional bands of 21.2 and 24.7 kb respectively, which agrees with the deletion of gII glycoprotein gene from BHV-1 FM genome.

### DETAILED DESCRIPTION OF THE INVENTION

The above described objectives of the present invention have been reached by an IBRV mutant which fails to produce any antigenic IBRV gII polypeptide as a result of a deletion or insertion or both in the IBRV gII gene, and a vaccine for IBR disease comprising: (1) a pharmaceutically effective amount of said virus and (2) a pharmaceutically acceptable carrier or diluent.

In the preferred embodiment of the present invention, the above described objectives have been reached by obtaining an IBRV which does not produce any antigenic gII polypeptide as a result of a deletion in the IBRV gII gene comprising:
(1) Constructing a first hybrid vector containing the DNA of the cloning vector and an IBRV DNA fragment containing 1200 bp of the 5' flanking region of the gII glycoprotein gene and 104 nucleotides of the sequence which codes for the same gene;
(2) Constructing a second hybrid vector containing the DNA of the cloning vector and an IBRV DNA fragment containing 1740 bp of the 3' flanking region of the gII glycoprotein gene and one nucleotide from the stop codon of the same gene;
(3) Constructing a third hybrid vector containing the DNA of the cloning vector and the IBRV DNA fragment from step (1) fused to the IBRV DNA fragment from step (2). This third hybrid vector bears a deletion of 1619 bp in the gII glycoprotein gene;
(4) Cotransfecting the hybrid vector from step (3) mixed with infectious gII⁺ IBRV DNA in the IBRV host cells;
   and,
(5) Screening the progeny viruses obtained in step (4) to identify and to obtain IBRV mutants which do not produce any antigenic IBRV gII polypeptide as a result of a deletion in the gII glycoprotein gene.

In a further embodiment of the present invention, a foreign DNA sequence is inserted in place of the IBRV gII gene sequence deleted in the step (3) in such a way that no antigenic IBRV gII polypeptides are produced and in such a way that IBRV DNA sequences adjacent to each side of the deleted IBRV gII gene sequences are retained. As a result, IBRV mutants of step (5) do not produce any antigenic IBRV gII polypeptide due to the combination of mutations by deletion and insertion in the IBRV gII gene.

In other embodiment form, the steps (1-3) were performed in order to insert a foreign DNA sequence into the plasmid in such a way that no antigenic IBRV gII polypeptides are produced, as well as retaining IBRV DNA sequences adjacent to each side of the insertion point. As a result, the IBRV mutant from step (5) does not produce any antigenic gII polypeptide due to an insertion in the IBRV gII gene.

The mutations of the IBRV gII gene of the present invention can be produced, for example, by: (a) deleting an IBRV DNA fragment in such a way that it eliminates or substantially alters the reading frame of the gII gene and the synthesis of the encoded polypeptide; (b) deleting the IBRV DNA to eliminate the nucleotide sequences encoding the epitopes of IBRV gII polypeptide; (c) inserting a foreign DNA sequence in the IBRV gII gene in such a way that it eliminates or substantially alters the reading frame of the gII gene; (d) deleting nucleotide sequences of the IBRV gII gene and at the same time inserting foreign DNA sequences, in such a way that it alters the reading frame of the gII gene and the synthesis of the encoded polypeptide; (e) creating point mutations in the sequence of IBRV gII gene in such a way that it alters the reading frame of the gII gene or the synthesis of the encoded polypeptide or the epitopes of IBRV gII polypeptide; (f) altering the expression of the IBRV gII glycoprotein gene by deletion, insertion or point mutation of the regulatory sequences of the IBRV gII gene in such a way that the gene is not transcribed.

The deletions may be produced in different ways, for example by: (a) digesting the IBRV gII gene inserted in a cloning vector with one or more restriction endonucleases to cut the IBRV gII gene at one or more sites, and then religating to remove the IBRV gII nucleotide sequences; (b) cutting the IBRV gII gene in a cloning vector at one site, followed by exonuclease treatment to remove 5' and/or 3' nucleotides to the cut and then religating.

In the present invention, the deletion mutant BHV-1 FM gII, described in detail below, was obtained by eliminating a 1619 bp Sma I-Eco NI fragment which contains substantially all the coding sequences of the IBRV gII gene (see Figure 7). The size of this deletion ensured that (a) no polypeptide with antigenic determinants able to elicit antibodies against the IBRV gII glycoprotein in vaccinated animals would be made, or able to react with antiserum raised against IBRV gII glycoprotein; and (b) a reversion to IBRV gII⁺ by a mutation is virtually impossible.

In other embodiment form, deletion or insertion mutants can contain a foreign DNA sequence in place or in addition of the deleted IBRV gII gene sequence. As used herein, a "foreign DNA sequence" means: (1) any DNA sequence which does not encode a gene, regardless of its origin, such as a viral, a prokaryotic or an eukaryotic sequence, or even a synthetic oligonucleotide noncoding sequence; (2) any DNA sequence which encodes a gene other than an IBRV gII gene; and (3) any IBRV DNA sequence which has been translocated from its normal location in the IBRV genome to another location on the IBRV genome. The length of the above mentioned foreign DNA sequences is not critical and their size can vary from a few nucleotides (i.e. 4) to genes of large size (i.e. thousands of base pairs). The way of inserting foreign DNA sequences is very variable, existing some well-known procedures (Sambrook et al., Molecular Cloning, Cold Spring Harbor Laboratory (1989). Frame shift mutations can be produced in the IBRV gII gene and they also give rise to a IBRV gII⁻.

Several cloning vectors are used to construct the hybrid plasmids comprising IBRV DNA fragments (IBRV gII gene and flanking sequences). The cloning vectors used in the present invention will be detailed below. The specific host used to clone the hybrid vectors is not critical to the present invention. The specific host used in the preferred form of the invention will be detailed below. Alternative vectors and specific hosts other than *E. coli* can be used (i.e. yeast) to perform the present invention.

IBRV gII gene sequences that must be present in the hybrid vectors of the previously described steps (1) to (3), will depend on the sequences chosen for deletion and/or insertion, and the restriction endonucleases and exonucleases to be used in the engineering of the deletion and/or insertion mutant. Both the IBRV DNA sequences adjacent to the deletion and/or insertion site in the vector required in the steps (1) and (2) and their length, will depend on the specificities of the deletion and/or insertion to be created. Those that were used will be detailed in the description of the preferred form of the invention.

The source of IBRV, IBRV gII gene and protein used in the present invention is not critical. The strain can be either non-temperature resistant or temperature resistant. In the present invention the specific IBRV strain used was a wild type strain isolate by Laboratorios HIPRA S.A. and named "FM" (hereinafter"BHV-1 FM").

IBRV host cell strains used in the present invention are not critical as long as they allow the permissive growth of IBRV. Those that were used will be detailed in the below description of the preferred form of the invention.

The method for screening for IBRV gII mutants in step (5) is not critical in the present invention. For example, the screening can be performed by dot-blot hybridization using either non-radioactive or radioactive DNA probes. These probes contain IBRV gII gene DNA sequences which would be expected to be absent from the IBRV gII deletion mutants, or foreign DNA sequences expected to be present in the IBRV gII insertion mutants and deletion/insertion mutants. The screening methods can be also performed with RNA probes. The screening for IBRV mutants in step (5) can also be done by methods other than those requiring nucleotide probes. For example, using monoclonal or monospecific antibodies against the IBRV gII glycoprotein, plaques can be screened by immunological methods for mutant viruses that do not express IBRV gII. Another possibility is to use polyclonal, monoclonal or monospecific antibodies against foreign IBRV protein expressed in the IBRV. In this way, IBRV gII deletion and/or insertion mutants can be identified and isolated.

The gII⁻ IBRV mutants described in the present invention can be used as a modified-live virus vaccine or as a killed virus vaccine against IBR disease. The inactivation of infectivity of the gII⁻ IBRV mutants is done by chemical or physical treatments (formaldehyde, ultraviolet light, etc.). Any of the vaccine types described above yields a vaccine capable of eliciting both humoral and cellular response in the immunized animal. These gII⁻ IBRV mutants can be exploited to obtain subunit IBRV vaccines, after extraction and purification of their glycoproteins by known methods. Alternatively, these gII⁻ IBRV mutants can be used as a starting material to obtain other IBRV mutants that exhibit additional deletions and/or insertions (for example, in thymidine kinase or other glycoproteins).

The virus described in the present invention can be used for the preparation of an efficient vaccine against IBR disease. To this purpose, an effective amount of virus is combined with a carrier or suitable diluent. The suitable diluents that can be used in the present invention are: silica salts, aluminium hydroxide, saponins (i.e. QuilA), calcium hydroxide, and other adjuvants whose relevant function is to increase the antigenicity of the vaccine components. In addition, gII⁻ IBRV mutants can be emulsified with mineral oils like: Markol® Drakeol® and others under different emulsified formulations (o/w, w/o/w and w/o).

The preferred storage conditions for the gII⁻ IBRV mutants of the present invention are either at a titer of 10⁵ to 10⁶ p.f.u./mL and below -70°C in 10% glycerol or lyophilized and stored at -4°C to -20°C. The lyophilized virus may be reconstituted with sterile-distilled water or with appropriate buffers, either with or without preservatives.

The useful dosage of the IBRV gII⁻ to be administered in the immunization process will vary depending on the age, weight, mode of administration and species. If used without inactivation, a suitable dosage in the vaccine can be about 10^{4.5} to 10⁷ p.f.u./animal, preferably about 10^{4.5} to 10^{5.5} p.f.u./animal. If used inactivated, a suitable dosage in the vaccine can be, for example, about five-fold to ten-fold than that employed before.

The vaccines that contain gII⁻ IBRV mutants described in the present invention can be administrated intramuscularly, intradermically, intranasally or subcutaneosly, being intramuscularly the preferred mode of administration.

The following example is provided for illustrative purposes of the development of the present invention, and it is in no way intended to limit the scope of the present invention.

### 4.- EXAMPLE OF THE PREFERRED MODE OF THE PRESENT INVENTION

The objectives of the present invention are related to the obtention of a mutant strain of BHV-1, by DNA deletion/insertion in the locus of IBRV gII glycoprotein gene. As a result, the virus will not produce or present the gII glycoprotein in its envelope. The invention includes the development of a BHV-1 virus strain that can be used as a vaccine against IBR. In addition, the present invention provides the tools for a differential-diagnostic method allowing the distinction between animals infected with any IBRV gII⁺ field strain, from animals vaccinated with the IBRV gII⁻ strain. The detailed description of invention (section 4th) is divided in the following sections:
**4.A.** to **4.G.** Mapping, cloning, sequencing and identifying the structural gene for IBRV gII glycoprotein.
**4.H.** to **4.J.** The construction of a hybrid vector containing 5' and 3' flanking sequences from the gII glycoprotein gene.
**4.K.** A method to delete the gII glycoprotein gene sequence and to construct a defective gII⁻ virus strain, by cotransfecting the hybrid vector pΔgII with DNA from BHV-1 FM (gII⁺) in host cells.
**4.L.** and **4.M.** A screening method of the progeny viruses, obtained as described in the step **4.K.**, to identify and isolate IBRV virus particles which have a mutation in the locus corresponding to gII glycoprotein gene .
**4.N.** Analyses of purified BHV-1 gII⁻ DNA to check that progeny viruses isolated in the step **4.M.** are homogeneous and have a deletion in the gII locus.
**4.O.** List of sequences.

### Sections 4.A. to 4.G. Mapping, cloning, sequencing and identifying the structural gene for IBRV gII glycoprotein.

### 4.A. Cell cultures and virus purification.

The cell lines MDBK (Mandi-Darby Bovine Kidney) and GBK (Georgia Bovine Kidney) were used for BHV-1 virus propagation and production; these host cell lines were supplied by Laboratorios HIPRA S.A. These host cells were grown as monolayer cultures in plastic flasks (i.e. Falcon) at 37°C (5% CO₂), in Glasgow modified Minimum Essential Medium Eagle (CULTEK S.A.), with 0.2% (w/v) tryptose phosphate broth, 0.5 mM sodium pyruvate and 0.1 mg/mL gentamycin (hereinafter "GMEM"), supplemented with 10% (v/v) fetal calf serum (hereinafter "FCS"). The cell lines were checked for the presence of adventitious viruses and mycoplasma.

The BHV-1 virus strain used in the present invention was isolated from an infectious outbreak field and named "FM" by Laboratorios HIPRA S.A. As described below, the restriction map of this strain is similar to the BHV-1 Cooper strain. The viruses were stored at -80°C in GMEM containing 10% (v/v) glycerol. The BHV-1 FM was amplified and propagated in MDBK monolayers using the medium and conditions described below. These cultures were infected at a multiplicity of infection (hereinafter "M.O.I.") of 0.1-0.01. More specifically, a desired virus dilution was made in GMEM supplemented with 1% FCS; the cultures were spread with 0.04 mL of virus dilution per cm² of cell monolayer and incubated for 2 h at 37°C. Then, GMEM with 1% FCS was added and the incubation was continued until an extensive cytopathic effect was observed (usually 48 h). Alternatively, after 2 h of adsortion the virus dilution was removed and the cell monolayers were spread with GMEM containing 1% FCS. The viruses produced by the method of infection above described were purified as follows: the culture medium was collected (30-35 mL per 175 cm² bottle) and centrifuged at 500-1000xg at 4°C for 20-30 min to eliminate cells and cellular debris. Next, the resulting supernatant was recovered and centrifuged for 3 h at 48000xg at 4°C to obtain a virus pellet. This pellet was stored as mentioned above or used to obtain viral DNA (see step **4.B.**). Alternatively, an additional step, based on a centrifugation of viruses in a sodium potassium tartrate density gradient, was added to the virus purification protocol; this step was as follows: the virus pellet was resuspended in 0.5 mL of a buffer containing 10 mM Tris-HCl (pH 8.0), 1 mM EDTA, 0.2 M NaCI (hereinafter "STE"), and this suspension was applied on to a discontinuous sodium potassium tartrate gradient: 20, 30, 40 and 50 % (w/v). Next, the gradient was centrifuged for 3h at 100000xg (26,500 rpm Beckman, SW-27 rotor) at 4°C. In this way, cellular membranes remain in the upper layer of the gradient, while intact virus particles sediment on the 50% layer. Nonenveloped viruses are pelleted under these conditions. After centrifugation, the band corresponding to the intact virus particles was recovered, diluted four times with STE and centrifuged for 3h at 48000xg at 4°C. Finally, the virus pellet was resuspended in 50 µL of STE.

The virus titulation was carried out by infecting cell monolayers in solid media. Cells were seeded in 90 mm Petri dishes with GMEM containing 10% FCS and cultured up to obtain a monolayer. Next, the medium was aspirated and the cell monolayer was infected with a virus dilution (10⁻⁴ to 10⁻⁸) in GMEM with 1% FCS, and incubated at 37°C for 2 h. Then, the media was removed and the cell monolayer was overlaid with GMEM with 1% FCS and 1% (w/v) low melting agarose (BioRad). Upon agarose solidification, Petri dishes were incubated at 37°C until infection areas were observed (usually, 3 days). It is noteworthy that extensive cytopathic effect promotes (among other morphologic alterations) the detachment of infected cells from the plastic surface, giving rise to tiny areas (plaques) without cells, which are easily seen. This technique allows the accurate titration of the p.f.u. in a virus sample. Alternatively, the plaques can be counted by staining the cell monolayer (i.e. Giemsa stain) after removing the agarose layer. Colorless plaques can be seen over a background of stained cells.

### 4.B. Purification of BHV-1 FM DNA

BHV-1 FM DNA was purified from viruses prepared as described in step **4.A.**. Viruses were resuspended in STE (2 mL per virus pellet from 30-35 mL of culture medium). Next, 100µl of 10% (w/v) SDS and 200 µg/mL of proteinase K (Boehringer Mannheim) were added to the viral suspension and incubated at 37°C for 1 h. Then, two phenol extractions with an equal volume of phenol-chloroform-isoamyl alcohol (25:24:1 v/v/v) were carried out. The solution was mixed and centrifuged at 1000-2000xg for 3 min. The aqueous phase was transferred to a new tube and mixed with an equal volume of chloroform-isoamyl alcohol (24:1 v/v) to eliminate phenol traces. Next, 0.1 volumes of 3 M sodium acetate (pH 5.2) were added to the aqueous phase, and the nucleic acids were precipitated by adding 2 volumes of ethanol. The solution was mixed and the precipitate was collected by decantation in a 1.5 mL microcentrifuge tube. The precipitated DNA was sedimented by centrifugation at 12000xg, washed twice with 70% ethanol and air dried. Then, the DNA was redissolved in 50 µL of 10 mM Tris-HCl (pH 8.0), 1 mM EDTA (hereinafter "TE"). DNA concentration was quantified by optical density at 260 nm and their purity was checked by spectrophotometry scan analysis (320-220 nm) and agarose gel electrophoresis (as detailed in section **4.C.**).

### 4.C. Restriction analysis of BHV-1 FM genome

For the genome restriction analysis, 0.4 µg of BHV-1 FM DNA (obtained as described in step **4.B.**) were digested separately with the enzymes Hind III and Eco RI (Boehringer Mannheim). The digests were electrophoresed in 0.6% (w/v) agarose gels with 40 mM Tris-acetate (pH 8.1)1 mM EDTA (hereinafter "TAE") at 1 V/cm for 16 hat room temperature. DNA was stained by soaking the agarose gels in TAE with 0.5 µg/mL ethidium bromide for 20 min, visualized on an long wave UV transilluminator and photographed. The digests of BHV-1 FM DNA with the restriction enzymes Hind III and Eco RI are show in Figure 1A. The obtained digestion patterns were compared with those reported in the literature. The restriction patterns of BHV-1 FM strain were very similar to those described for the Cooper strain (Mayfeld, J. E. et al. (1983) J.Virol., 47: 259-264; Whetstone, C. A. et al. (1989) Arch. Virol., 106: 261-279 ) (Figure 1B). The Hind III digestion pattern presents a band of 8.4 kb, corresponding to a fragment which includes most of the BHV-1 Us region, as elsewhere reported (Whetstone, C. A. et al. (1989) Arch. Virol., 106: 261-279; Simard, C. et al. (1990) Arch. Virol., 110: 63-75, Bulach and Studdert, (1990) Arch. Virol., 17-34). This data would indicate that the BHV-1 gII glycoprotein gene is located in this fragment. In the same way, the 16.2 kb band from Eco RI digestion includes the entire BHV-1 Us region. This fragment may also conatin the BHV-1 gII glycoprotein gene.

According to the starting hypothesis of the present invention, the BHV-1 gII glycoprotein gene would be located in the Hind III K band (Figure 1). This hypothesis agrees with the reported data on other members of the *Alphaherpesviridae* family, which presents a sequence of five glycoproteins that are located in conserved positions through the genome: US4 (gG), US5, US6 (gD), US7 (gI) and US8 (gE) from **HSV-1**; US4 (gG2), US5, US6 (gD2), US7 (gI2) and US8 (gE2) from **HSV-2**; gX, gp50, gp63 and gI from **PRV** and gpIV and gpI from **VZV** (McGeoch, D. J. (1990) J.Gen. Virol., 71: 2361-2367 ). Therefore, the reported data strongly suggest that the BHV-1 gII glycoprotein gene is located in the Us region of the genome.

### 4.D. Cloning of the BHV-1 FM DNA

The Hind III fragments of BHV-1 FM DNA were cloned at the Hind III site of pTZ1 8u (United States Biochemical, Corp.), by the following procedure:
2 µg of BHV-1 FM DNA were dissolved in a buffer containing 10 mM Tris-HCl (pH 8.0), 5 mM MgCl₂, 100 mM NaCI and 1 mM mercaptoethanol (hereinafter "Hind III digestion buffer"). The DNA was digested at 37°C for 1 h with 2 units of Hind III. The restriction enzyme Hind III was inactivated by heating at 65°C for 20 min. The resulting DNA fragments were mixed with Hind III digested pTZ18u DNA by the following method:
2 µg of Hind III digested BHV-1 FM DNA were mixed with 100 ng of Hind III digested pTZ18u, in 20 µl of a buffer containing 66 mM Tris-HCl (pH 7.5), 5 mM MgCl₂, 1 mM dithiothreitol, 1 mM ATP (hereinafter "ligation buffer"), 2 units of phage T4 DNA ligase (Boehringer Mannheim), and were incubated overnight at 16°C.

The resulting hybrid plasmids were used to transform *E. coli* XL1-Blue as described below:
Competent *E*. *coli* XL1-Blue were prepared for transformation using the CaCl₂ procedure described by Sambrook et al. (Sambrook et al. Molecular Cloning, (1989) Cold Spring Harbor Laboratory). In a detailed form, a 1 mL overnight culture of *E. coli* XL1-Blue was used to inoculate 200 mL of broth containing 1.6% (w/v) bactotryptone, 1% (w/v) yeast extract and 0.5% (w/v) NaCI (hereinafter "2xYT broth"). The culture was incubated at 37°C for 6-7 h at 300 rpm. The bacteria were collected by centrifugation and resuspended in 1/2 volume of ice cold 0.1 M CaCl₂. After a 5 min incubation on ice, the bacteria were pelleted and resuspended in 1/15 of the initial culture volume of ice cold 0.1 M CaCl₂.

Next, 20 µl of the hybrid plasmid DNA was added to 0.2 mL of the CaCl₂- competent bacteria. The mixture was kept on ice for 30 min, incubated for 90 sec at 42°C, and then 0.5 mL of 2xYT broth was added. The mixture was incubated at 37°C for 1h with gentle shaking. Samples were plated on Petri dishes with 20 mL of solid media containing 1% (w/v) tryptone, 0.55% (w/v) yeast extract, 1% (w/v) NaCI (hereinafter "LB") and 1,5 % bactoagar, supplemented with 60 µg/mL ampicillin, 0.04 mg/mL S-bromo-4-chloro-3-indolyl-β-D-galactopyranoside (hereinafter "X-gal") and 0.04 mg/mL isopropyl-β-D-thiogalactopyranoside (hereinafter "IPTG").

Rapid screening for the hybrid plasmid DNA (hereinafter "miniprep") was made as follows:
Each recombinant clone was inoculated in 5 mL 2xYT broth supplemented with 60 µg/mL ampicillin. The cultures were incubated overnight at 37°C with shaking (300 rpm). One mL of each overnight culture was centrifuged to pellet the bacteria. The bacterial pellet was resupended in 0.2 mL of a buffer containing 50 mM glucose, 25 mM Tris-HCl (pH 8.0), and 10 mM EDTA (hereinafter "solution I"). Thereafter, 0.4 mL of solution containing 0.2 N NaOH and 1% (w/v) sodium dodecylsulfate (hereinafter "SDS") (hereinafter "solution II") were added to each sample and the tubes were gently mixed and kept for 5 min at room temperature. Next, 0.3 mL of solution containing 3M potassium acetate and 2M acetic acid (hereinafter "solution III") were added to each sample and the tubes were gently inverted, kept on ice for 5 min and centrifuged for 10 min at 13000xg. The resulting supernatant was transferred to a new tube and, then, 1 volume of phenol-chloroform-isoamyl alcohol (25:24:1 v/v/v) was added. The mixtures were vigorously shaked and centrifuged for 5 min at 13000xg. The aqueous phase was transferred to a new tube and the phenol extraction was repeated. Then, 2.5 volumes of ethanol were added and the mixture was placed at -20°C for 30 min. The precipitated hybrid plasmid DNA was collected by centrifugation at 13000xg for 10 min. and rinsed with 70% (v/v) ice cold ethanol and air dried. Finally, the plasmid DNA was dissolved in 200 µL of TE buffer.

One µL of 10xHind III cutting buffer and 2 units of Hind III were added to 9 µL-aliquots of hybrid plasmid DNA from each clone. Upon 3 h incubation at 37°C, samples were mixed with 1/10 volume of a solution containing 0.4% (w/v) bromophenol blue, 125 mM EDTA, and 50% (v/v) glycerol. The resulting solution was applied to a 0.6% (w/v) agarose slab gel for electrophoretic analysis. This analysis showed those hybrid plasmids containing a Hind III insert from BHV-1 FM DNA and its size. An 11.26 kb hybrid plasmid DNA containing an 8.4 kb insert, which comigrated with BHV-1 FM Hind III K fragment, was isolated and named p1K1 (Figure 2).

For large scale preparation of hybrid plasmid DNA, a 100-fold amount of bacteria was processed respect to the miniprep procedure above described, with the exception that the DNA was precipitated with 0.6 volumes of isopropanol from the supernatant obtained after the addition of solution III. Thereafter, the sample was kept for 5 min at room temperature and then centrifuged at 13000xg for 10 min. The precipitate was dissolved in 3 mL of TE buffer and mixed with 3 g of CsCI and 0.4 mL of 10 µg/mL ethidium bromide. The mixture must have a density of 1.55g/mL and a refractive index of 1.3860. The solution was transferred to a centrifuge tube and centrifuged at 65000 rpm for 16 h at 20°C in a Beckman VTi80 rotor. The result of the centrifugation was the presence in the middle of the tube of a red fluorescent band corresponding to the supercoiled DNA from the hybrid plasmid. The hybrid plasmid was collected by puncturing the tube with a needle. To remove ethidium bromide, the recovered DNA was mixed with 1 volume of water-saturated 1-butanol; the two phases were mixed and centrifuged at 13000xg for 30 s at room temperature. The upper phase containing the ethidium bromide was removed. Such extraction was repeated 8 times until the upper phase did not contain any fluorescent trace. Next, to precipitate DNA 3 volumes of distilled water and 2 volumes of ice cold ethanol were added to the lower phase. The solution was centrifuged at 10000xg for 15 min. The hybrid plasmid DNA pellet was dissolved in an adequate volume of TE buffer. DNA concentration was quantified by optical density at 260 nm and their purity was checked by spectrophotometry scan analysis (320-220 nm) and agarose gel electrophoresis (as detailed in section **4.C.**).

### 4.E. Mapping of the structural BHV-1 FM gII gene

In order to locate the structural gII gene into the Hind III K band from BHV-1 genome, it was necessary to determine the position of restriction enzyme sites. This was done as follows:

The p1K1 plasmid was linearized with Eco RI restriction enzyme, which did not cut the Hind III K insert. Subsequently, the linearized p1K1 plasmid was digested with the following enzymes: Sal I, Xho I, Pat I and Sma I, in suboptimal conditions respect to those recommended by the supplier and at different times to obtain partial digestions of the Hind III K fragment inserted in the p1K1 hybrid plasmid. The digests were electrophoresed in an agarose gel, Southern blotted and hybridized with pUC118 previously linearized with EcoR I and labeled with digoxigenin (hereinafter "DIG") by random primed technique (as detailed in section **4.F.**). The hibridization showed a ladder of bands representing the restriction sites in the insert of the hibrid plasmid p1Ki. With this information and knowing the size of the fragments obtained in a complete digestion of p1K1 hybrid plasmid DNA with the above described restriction enzymes, it was possible to construct a detailed restriction map of the 8.4 kb insert cloned in the plasmid p1K1 (Figure 3). This was done in the following way:
Digests were electrophoresed in a 1% (w/v) agarose gel in TAE buffer for 16 h at room temperature at 16V. The DNA was stained with 0.5 µg/mL ethidium bromide in TAE buffer and visualized on a long wave UV transilluminator and photographed. Next, the gel was soaked in 1 M NaOH for 30 min at room temperature and then soaked in 1 M Tris-HCI (pH 7.0), 1.5 M NaCI for 60 min at room temperature. Thereafter, the gel was capillary blotted on a nitrocellulose sheet (Schleicher and Schuell), previously hydrated and soaked in 3M NaCI and 0.3 M sodium citrate (hereinafter "20xSSC"). Blotting was performed overnight at room temperature with 10xSSC as transfer buffer. The filter was rinsed with 6xSSC, air dried at room temperature and baked in a vacuum oven at 80°C for 2 h. Subsequently, the nitrocellulose filter was placed in a plastic bag and soaked with 20 mL of 5x(0.1% (w/v) Ficoll, 0.1% (w/v) polyvinylpirrolidone and 0.1% (w/v) bovine serum albumin) (hereinafter "Denhardt's solution"), 5xSSC, 50% (v/v) formamide and 100 µg/mL single stranded DNA (hereinafter "prehybridization solution"). The single stranded DNA was prepared as follows: 50 mg of salmon sperm DNA was dissolved in 10 mL of 0.2 M NaOH, denatured by heating at 100°C for 20 min, sheared to about 0.5 kb fragments by pumping with a syringe, and finally neutralized with 0.2 mL of 10 M HCl. The nitrocellulose filter was incubated for 2 h at 42°C; and meanwhile, the DIG-labeled probe (section **4.F.**), previously heated at 100°C for 10 min and cooled rapidly in ice, was added to a solution containing 5xSSC, 45% (v/v) formamide, 5x Denhardt's solution and 7.5% (w/v) dextran sulfate (hereinafter "hybridization solution"). Next, 3 mL of hybridization solution containing 50-100 ng of denatured DIG-labeled probe were added per 100 cm² nitrocellulose filter and left to hybridize overnight at 42°C. Thereafter, the filter was washed twice for 15 min at 42°C with 2xSSC and 0.1% (w/v) SDS solution, and then washed twice for 15 min at 68°C with 0.1xSSC and 0.1% (w/v) SDS solution. Subsequently, the filter was rinsed with 100 mM Tris-HCl (pH 7.5) and 150 mM NaCI (hereinafter "buffer 1") and then incubated for 30 min at room temperature with 100 mL of 0.5% (w/v) blocking reagent in buffer 1 (Boehringer Mannheim) (hereinafter "buffer 2"). The nitrocellulose filter was briefly rinsed with buffer 1 and incubated for 30 min at room temperature with 20 mL of buffer 2 containing a dilution1:5000 of anti-digoxigenin conjugated to alkaline phosphatase (Boehringer Mannheim). Next, the nitrocellulose filter was briefly rinsed twice for 15 min with buffer 1 and equilibrated for 2 min with 20 mL of 100 mM Tris-HCI (pH 9.5), 100 mM NaCI and 50 mM MgCl₂ (hereinafter "buffer 3"). Finally, nitrocellulose filter was incubated with 10 mL of buffer 3 containing 45 µL of 75 mg/mL nitroblue tetrazolium in 70% (v/v) dimethylformamide and 35 µL of 50 mg/mL 5-bromo-4-chloro-3-indolyl phosphate, toluidinium salt in 100% (v/v) dimethylformamide (hereinafter "developing buffer"). Once the desired bands were detected, the developing reaction was stopped by washing the nitrocellulose filter with 50 mL of TE.

### 4.F. Preparation of probes for molecular hybridization

The starting material was linearized-DNA previously purified by phenolchloroform and precipitated with ethanol. The DNA was denatured by heating at 95°C for 10 min and quickly ice-cooled. The labeling reaction was prepared by adding in an ice-cold microcentrifuge tube: 1 µg of denatured DNA, 2 µL of an hexanucleotide mixture (Boehringer Mannheim), 2 µL of dNTP labeling mixture (1mM ATP, 1mM CTP, 1mM GTP, 0.65 mM TTP, 0.35 mM DIG-dUTP (pH 6.5)), 1 µL of Klenow enzyme (2U/µL) and distilled water to complete a final volume of 20 µL. The tube was incubated at 37°C for 20 h. The reaction was stopped by adding 2 µL of 0.2 M EDTA (pH 8,0). The labeled DNA was precipitated by adding 2.5 µL of 3 M sodium acetate (pH 5) and 75 µL of cold ethanol. The mixture was kept at -80°C for 30 min, and then centrifuged at 12000xg for 10 min. The DNA pellet was washed with 70% (v/v) ethanol, vacuum-dried and redissolved in 50 µL of TE.

### 4.G. Sequencing of the BHV-1 FM gII gene.

In order to determine the accurate location of the gene coding for the gII glycoprotein, two fragments from the insert of hybrid plasmid p1K1 according to the previously obtained restriction map were subcloned. These fragments were subcloned into the pUC118 plasmid upon digestion of the plasmid p1K1 with the restriction enzymes Sal I and Pst I, and isolated by 1% (w/v) agarose-gel electrophoresis, as above described. The bands of interest were eluted from the gel by using the Geneclean (Bio 101) system, according to the following procedure:

After electrophoresis in TAE buffer, the agarose gel was stained with ethidium bromide. DNA bands were visualized by long wave UV-light and agarose fragments including the DNA bands of interest were excised. These fragments were placed in a microfuge tube and 2.5 volumes (respect to the weight of agarose fragments -mg/mL-) of 6M Nal solution were added. The microfuge tube was incubated at 55°C for 5 min to melt the agarose. Then, 5 µL of glassmilk suspension (Bio 101) were added to the microfuge tube and kept for 5 min on ice. The tube was briefly centrifuged at 13000xg and the supernatant carefully removed. Next, the pellet was resuspended in 0.7 mL of washing solution (Bio 101), centrifuged at 13000xg for 10 sec and the supernatant discarded; this step was repeated twice more. Finally, the pellet was resuspended in 10 µL of TE buffer and the suspension incubated at 55°C for 5 min, centrifuged at 13000xg for 10 sec and the supernatant recovered. The DNA so obtained can be used directly or further purified by ethanol preciptation.

The subcloned fragments were those denoted as Sal IA of 1095 bp and Pst IC of 1654 bp (Figure 3). Then, a second subcloning step was carried out to obtain smaller fragments suitable for sequencing.

The Sal IA fragment, previously purified by Geneclean, was digested with the following restriction enzymes: Pst I to obtain the subclones Sal A1, Sal A4, and Sal A5; Sma I to obtain the subclones Sal A2 and Sal A3; and, Sac I and Pst I to obtain the subclones Sal A31 and Sal A33 (Figure 5).

The Pst IC fragment, previously purified by Geneclean, was digested with the following restriction enzymes: Sma I to obtain the subclones Pst C1 and Pst C2; and, Sac I to obtain the subclone Pst C3 (Figure 5).

The hybrid plasmid DNA of each subclone was obtained from overnight 3mL cultures by the procedure described for the miniprep procedure and further purified by Geneclean.

Sequencing reactions were carried out following the usual dideoxy chain termination method (Sanger, F. et al. (1977) Proc. Natl. Acad. Sci. USA 74; 5463-5467). The reaction mixture contained: an alkali-denatured template corresponding to each one of the above described subclones; the "universal" and "reverse" oligonucleotides corresponding to pUC118 vector were used as primers for DNA synthesis, (α-³⁵S)dATP as labeling substrate, Mg²⁺, deoxyribonucleosides triphosphate, dideoxyribonucleosides triphosphate and T7 DNA polymerase (according to the directions from Pharmacia). The reaction was stopped by adding a solution containing 0.3% (w/v) bromophenol blue, 0.3% (w/v) xylene cyanol, 10 mM EDTA (pH 7.5) and 97% (v/v) deionyzed formamide. Reactions were heated at 90°C for 1 minute and loaded onto a 8% (w/v) sequencing gel, composed by 7.7% (w/v) acrylamide, 0.3% (w/v) bisacrylamide, 32% (w/v) urea, 0.003% (v/v) TEMED and 0.007% (w/v) ammonium persulfate.

The analysis of the obtained sequences showed a high degree of identity between the sequence corresponding to the Sal IA fragment and those corresponding to the 5' end of the g1 (PRV), gE (HSV-1), and gE (EHV-1) genes.

The sequence obtained from the 3' end of the Hind III K fragment does not contain the whole sequence coding for the BHV-1 FM gII glycoprotein. In order to obtain the complete 3' end of the gene, a strategy of "walking" along the Us sequence of the viral genome was used. For this purpose, the BHV-1 FM DNA was digested with a series of restriction enzymes having restriction sites into the Hind III K fragment and, therefore, they should produce fragments beyond the 3' end of the Hind III K fragment. The following enzymes were used: Hind III (as a control), Xho I, Sal I, Sma I, Eco RI/Hpa I, Nco I, Bst EII, and Sac I. The viral DNA digests were electrophoresed in 0.8% agarose gels, blotted to nitrocellulose filters and hybridized to the DIG-labeled Sal 1A fragment under high stringent conditions as above described. The fragments hybridizing to Sal 1A fragment were used to construct a restriction map for the further cloning and sequencing beyond the Hind III K 3' region. The results of such hybridization are shown in Figure 4.

The results from Southern blot made evident the existence of a Sal I-Eco RI fragment (5.2 kb) overlapping the 3' end of the Hind III K fragment.

Taking into account from these results, it was first decided to isolate the Eco RI C fragment (Figure 1A and B). For this purpose, the viral genome was digested with Eco RI/Hpa I and electrophoresed in a 0.5% (w/v) agarose gel for 48 h in order to resolve the Eco RI C fragment. The additional Hpa I digestion was carried out to remove an undesired 15 kb band which comigrates with the Eco RI C band. This band was withdrawn from the gel and the DNA purified by Geneclean. The resulting fragment was further digested with Sal I to clone the sequence including the 3' end of the BHV-1 FM gII gene. The hybrid plasmid DNA containing the 5.2 kb Sal I-Eco RI insert is herein named pSE1.

Forthwith, a restriction map of pSE1 was obtained according to the methods above described and the fragments closer to the 5' end of pSE1 were subcloned by using the following restriction enzymes: Nhe I, Pst I, Sac I, and Xho I (Figure 5).

The 2400 bp Pst I-Pst I fragment was obtained from the Sal I-Eco RI fragment inserted into the hybrid plasmid pSE1, and subcloned into pUC118 to generate the hybrid plasmid pSE10. The Pst I-Pst I fragment was digested with: Xho I to obtain the subclones pSE13 and pSE14; Sac I to obtain the subclone pSE12; Sac I and Nhe I to obtain the subclone pSE121; and, Acc I to obtain the subclone pSE141 (Figure 5).

These fragments were sequenced as above described. The complete nucleotide sequence of BHV-1 FM gII gene is shown in the section **4.O.**. Table 1 shows the restriction sites predicted from the sequence.

The nucleotide sequence contains an open reading frame (575 codons) coding for a polypeptide with a molecular mass of 65 kDa.

The translation start codon (ATG) is found 20 nucleotides downstream the first BstB I site. The reading frame of 1725 nucleotides is delimited by a stop codon (TAG), which is located into the Eco NI site (Figure 5 and Table 1). No evidence about the existence of a polyadenylation signal downstream the reading frame has been found. This feature is also observed in the homologous genes from HSV-1 and EHV-1, where the polyadenylation signal is shared by the gII homologous gene and the contiguous gene (McGeoch, D. J. et al. (1985) J. Mol. Biol., 181; 1-13; Telford, E. et al. (1992) Virology, 189; 304-316). A TATA transcription signal has been found 131 nucleotides upstream the start codon. This sequence differs slightly from the consensus (Bucher, P. (1990) J. Mol. Biol., 212; 563-578), although it has also been described for the BHV-1 tk gene (Bello, L. J. et al. (1992) Virology, 189; 407-414) and for various genes of the HSV-1 Us region (McGeoch, D. J. et al. (1985) J. Mol. Biol., 181; 1-13; Bucher, P. (1990) J. Mol. Biol., 212; 563-578). Two GC boxes, related with transcription efficiency, have been found at positions -169 and -226 (Bucher, P. (1990) J. Mol. Biol., 212; 563-578). No CCAT sequence has been found upstream the TATA box, nevertheless the absence of a CCAT box has already been reported for most of the genes from the HSV-1 Us region (McGeoch, D. J. et al. (1985) J. Mol. Biol., 1-13). Finally, a cap box, related with the RNA polymerase II (Bucher, P. (1990) J. Mol. Biol., 212; 563-578), has been identified at position -102.

**TABLE 1**

| RESTRICTION SITES PREDICTED FROM THE gII GENE SEQUENCE OF BHV-1 (FM) | |
|---|---|
| Restriction endonucleases | Position of the first nucleotide into the sequence |
| | (Nucleotide no.) |
| Acc I | 507,1083,1425,1785 |
| Bst BI | 370,2214 |
| Eco NI | 2111 |
| Hind III | 1504 |
| Pst I | 924,1122,1482 |
| Sac I | 728 |
| Sal I | 507 |
| Sma I | 491,1091 |

The analysis of the amino acid (hereinafter "aa") sequence, predicted from the described reading frame, shows that the polypeptide displays all of the essential features of a membrane glycoprotein. Kyte-Doolittle algorithm predicts the existence of two hydrophobic regions consistent with sequences generating hidrophobic α-helices. The first domain expands from aa 11 to aa 31, and contains a protease recognition sequence between aa 26 and 27 (von Heinje, G. (1986) Nucleic Acids Res., 4683-4690). These data suggest the presence of a signal peptide from aa 1 to aa 26. This signal peptide presents, according to the model of Perlman, D. and Halvorson, H. O., 1983 (J. Mol. Biol., 167: 391-409): a) a N-terminal region with charged aa residues (3 arginines); b) an hydrophobic-rich aa central region (9 leucines and one phenylalanine over 14 residues) and c) a C-terminal region with short side-chains aa (2 alanines), which includes the processing site of the peptide. The signal peptide would allow the transport of the protein to the endoplasmatic reticulum where protein glycosylation occurs. The second hydrophobic region expands from aa 413 to aa 443. Its location close to the C-terminal end, together with the strong tendency of the segment comprised between the aa 422 and aa 442 to form a hydrophobic α-helix (von Heinje,-G. (1992) J. Mol. Biol., 255; 487-494; Sipos, L. and von Heinje, G. (1993) Eur. J. Biochem, 213; 1333-1340), make this region a good candidate to define a transmembrane domain. All of these features, and the presence of two consensus sites of N-glycosylation (sequence 141:AQLGR **N**AT GVLIA, and sequence 343:HLRPA **N**NS VDLVF; see section **4.O**.), indicate that the polypeptide coded by the gene object of the present invention is a glycoprotein.

Sequence homology analysis was performed by the FastA program (GCG Sequence Analysis Software Package; Madison, Wisconsin, USA) on a VAX/VMS system; such a program uses the Pearson-Lipman algorithm to search for similarities between a given sequence and a sequence data bank. The sequences of the different proteins used to perform the analysis were obtained from the GeneBank. As a whole, the sequence herein obtained shows a high identity degree (around 60%) versus protein sequences putatively homologous to the gII from other herpesviruses (gI from PRV; gE from HSV-1; and, gE from EHV-1).

**TABLE 2**

| | No. of aas | Mol. mass (Da) | % in number | | | |
|---|---|---|---|---|---|---|
| | | | Alanine | Glycine | Leucine | Proline |
| gII (BHV-1) | 573 | 61474 | 15.0 | 7.2 | 9.8 | 11.0 |
| g1 (PRV) | 576 | 62173 | 11.8 | 8.2 | 8.9 | 10.8 |
| gE (EHV-1) | 550 | 61143 | 6.2 | 5.3 | 9.6 | 7.8 |
| gE (HSV-1) | 550 | 59056 | 10.9 | 6.9 | 7.5 | 11.8 |

It is also remarkable the high content of proline residues (63 prolines: 15% in number and 9.9% of the molecular mass) in the present aa sequence (Table 2). This is a typical feature, not only of other homologous herpesviruses glycoproteins, but also of the other BHV-1 glycoproteins described in the literature (gI: 6.5%, gIII: 12.3%, and gIV: 12.5%). On the other hand, the aa sequence, not only has a similar proline content than EHV-1 gE (the most similar between the reported glycoproteins), but also conserves the positions in which prolines occur. Thus, from the 63 prolines, 19 occur at the same position (31.7%), 5 occur in similar positions (8.3%), 13 represent very conservative changes (21.7%), and 23 occur in different positions (38.3%), five of them inside the signal peptide sequence.

In the same way, the analysis of the obtained aa sequence with the homologous glycoproteins shows very high identity values: 32.5% identity with EHV-1 gE in a 532 aa overlap; 33.4% identity with PRV g1 in a 386 aa overlap, and 24.5% identity with HSV-1 gE in a 502 aa overlap. Therefore, we can conclude that the sequenced gene of the herein invention corresponds to the complete sequence of the gII glycoprotein from BHV-1 FM.

### Sections 4.H. to 4.J.: Design of a hybrid cloning vector containing the 5' and 3' flanking sequences of the gene coding for the gII glycoprotein.

### 4.H. Construction of the hibrid plasmid pC1.

The object of this step was to select a 1200 bp fragment corresponding wich includes an upstream region of the BHV-1 FM gII gene and the sequence wich codifies the signal peptide of the same gene. Both sequences are contained in the p1K1 insert.

One µg of p1K1 was digested for 5 h at 37°C with 2 units of Pst I and Sma I in 20 µL of a buffer containing 33 mM Tris-acetate (pH 7.9), 10 mM MgCl₂, 66 mM potassium acetate, and 0.5 mM dithiothreitol. After electrophoresis in 1% (w/v) agarose gel, a 1200 bp Pst I-Sma I fragment was purified by Geneclean as described above. It was also prepared pUC118 digested with Pst I and Sma I using the same conditions. Both DNAs were ligated with T4 ligase, and the resulting product used to transform *E. coli* XL1-Blue made competent with CaCl₂ treatment, as described above. The screened colonies contained a plasmid with a 1200 bp DNA insert. This hybrid plasmid was named pC1 (Figure 6).

### 4.1. Construction of hybrid plasmid pC1SE1a.

The objective of this step was to select a hybrid plasmid DNA containing a 4800 bp fragment composed by: (1) a 1200 bp DNA fragment corresponding to the upstream region of the BHV-1 FM gII gene, and the sequence correponding to the signal peptide from BHV-1 FM gII gene, fused to (2) a 3600 bp DNA fragment corresponding to the downstream region of the BHV-1 FM gII gene. This sequence also includes the STOP codon from BHV-1 FM gII gene.

Two µg of pSE1 were digested for 5 h at 37°C with 2 units of Eco NI (BioLabs) in 20 µL of a buffer containing 50 mM potassium acetate, 20 mM Tris-acetate (pH 7.9), 10 mM magnesium acetate, and 1 mM dithiothreitol. The digest was subjected to 1% (w/v) agarose gel electrophoresis, the band corresponding to the linearized plasmid was excised from the gel and finally purified by Geneclean. Then, the sticky ends of the linearized plasmid were blunt ended by Mung Bean nuclease (Boehringer Mannheim). Thus, the linearized pSE1 plasmid was incubated for 1 h at 25°C with 10 units of Mung Bean nuclease in 100 µL of a solution containing 20 mM sodium acetate (pH 4.6), 5 mM sodium chloride, 1 mM zinc acetate, and 0.001% (v/v) Triton X-100. After this treatment, 0.1 volumes of 3 M sodium acetate were added to the linearized pSE1 plasmid, which was then extracted with an equal volume of phenol/chloroform/isoamylalcohol (25/24/1 v/v/v) and precipitated with 2 volumes of ethanol. The redissolved plasmid was digested for 3 h at 37°C with 2 units of Eco RI in 20 µL of a buffer containing 50 mM Tris-HCl (pH 7.5), 10 mM MgCI₂, 100 mM sodium chloride, and 1 mM dithiothreitol. A 3600 bp Eco Nl(blunted)-Eco RI fragment from BHV-1 FM was electrophoresed in 1% (w/v) agarose gel, excised from de gel and purified by Geneclean.

At the same time, 2 µg of pC1 were digested for 3 h at 25°C with 2 units of SmaI in 20 µL of a buffer containing 33 mM Tris-acetate (pH 7.9), 10 mM MgCl₂, 66 mM potassium acetate, and 0.5 mM dithiothreitol. The solution was then adjusted to a final concentration of 100 mM sodium chloride in a volume of 40 µL, and was incubated for 2 h at 37°C in the presence of 2 units of Eco RI. The 4.4 kb resulting fragment was isolated by electrophoresis in 1% (w/v) agarose gel and purified by Geneclean. The resulting DNA fragment presented a Sma I (blunt end) and an Eco RI (sticky end).

Finally, the 3.6 kb Eco Nl(blunted)-Eco RI fragment from BHV-1 FM and the 4.4 kb Sma I-Eco RI fragment from pC1 were mixed and ligated with T4 ligase. The ligation product was used to transform competent *E. coli* XL1-Blue as above described. The screened colonies contained a plasmid with a 4.8 kb DNA insert being the size of the total hybrid plasmid DNA of 8 kb, corresponding to the fusion of pC1 plasmid (4.4 kb) with the 3.6 kb Eco Nl(blunted)-Eco RI fragment. This plasmid was named pC1SE1a (Figure 6).

### 4.J. Construction of the hybrid plasmid pΔgII.

The object of this step was to select a hybrid plasmid DNA containing a 3 kb fragment composed by: (1) a 1200 pb DNA fragment corresponding to the upstream region of the BHV-1 FM gII gene, and the sequence corresponding to the signal peptide from BHV-1 FM gII gene, fused to (2) a 1.8 kb DNA fragment corresponding to the downstream region of the BHV-1 FM gII gene, This sequence also includes the STOP codon from BHV-1 FM gII gene.

Two µg of pC1SE1a were digested for 3 h at 37°C with 2 units of Pst I in 20 µL of a buffer containing 50 mM Tris-HCl (pH 7.5), 10 mM MgCl₂, 100 mM sodium chloride, and 1 mM dithiothreitol. The digestion was then electrophoresed in 1% (w/v) agarose gel and the resulting 3.0 kb Pst I-Pst I fragment was purified by Geneclean as above described.

Two µg of pUC118 were digested for 3 h at 37°C with 2 units of Pst I in 20 µL of a buffer containing 50 mM Tris-HCI (pH 7.5), 10 mM MgCl₂, 100 mM sodium chloride, and 1 mM dithiothreitol. After digestion, 0.1 volumes of 3 M sodium acetate were added to the linearized pUC118 plasmid, which was then extracted with an equal volume of phenol/chloroform/isoamylalcohol (25/24/1 v/v/v) and precipitated with 2 volumes of ethanol.

The 3.0 kb Pst I-Pst I DNA fragment and the linearized pUC118 plasmid were mixed and ligated with T4 DNA ligase, and the ligation product used to transform competent *E. coli* XL1-Blue, as above described. The screened colonies contained a plasmid with a 3.0 kb DNA insert. This plasmid was named pΔgII (Figure 6).

### Section 4.K.: A method to delete the gII glycoprotein gene sequence and to construct a defective gII⁻ virus strain, by cotransfecting the hybrid vector pΔgII with IBRV DNA from BHV1 FM (gII⁺) in host cells.

The BHV-1 FM gII⁻, which lacks the ability of producing any antigenic polypeptide from gII, was obtained through a deletion in the gII gene from BHV-1 FM. This deletion was obtained by homologous recombination between the hybrid plasmid pΔgII and the genomic BHV-1 FM DNA. For this proupose both DNAs were cotransfected by electroporation of GBK cells.

Purified pΔgII was obtained by centrifugation in CsCl as described in section **4.D.**. Ten µg of pΔgII were digested for 3 h at 37°C with 10 units of Pst I in 100 µL of a buffer containing 50 mM Tris-HCl (pH 7.5), 10 mM MgCl₂, 100 mM sodium chloride, and 1 mM dithiothreitol. After digestion, 0.1 volumes of 3 M sodium acetate were added to the DNA, which was then extracted with an equal volume of phenol/chloroform/isoamylalcohol (25/24/1 v/v/v) and precipitated with 2 volumes of ethanol. The resulting DNA pellet was redisolved in 20 µL of sterile TE buffer.

BHV-1 FM gII⁺ DNA was obtained according to the procedure described in section **4.B.**.

GBK cells were prepared for electroporation as follows: 5.8x10⁶ GBK cells and 35 mL of GMEM supplemented with 10% FCS were added per 175 cm² culture flask. When a 60-70% confluent cell monolayer was obtained (72 h), the medium was removed and the cells rinsed with a buffer containing 0.04% (w/v) potassium chloride, 0.68% (w/v) sodium chloride, 0.22% (w/v) sodium bicarbonate, 0.014% (w/v) sodium dihydrogenphosphate, 0.1% (w/v) D-glucose, and 0.02% (w/v) EDTA (hereinafter, "EBSS-EDTA"). The EBSS-EDTA was removed and the cells detached from each culture flask by 3-5 min treatment with 3.5 mL of a solution containing 0.02% (w/v) EDTA and 0.05% (w/v) trypsin. Trypsin was inhibited by adding 3.5 mL of GMEM. Then, the cell suspension was collected and centrifuged at 800xg for 10 min at 4°C. After removing the supernatant, the cell pellet was resuspended in 20 mL of a buffer (cooled at 4°C) containing 20 mM HEPES, 137 mM sodium chloride, 5 mM potassium chloride, 0.7 mM disodium hydrogenphosphate, and 5 mM D-glucose (pH 7.1) (hereinafter, "saline HEPES"), and centrifuged at 800xg for 10 min at 4°C. The supernatant was removed and the cell pellet washed twice with saline HEPES in the conditions above described. Finally, the cell pellet was resuspended in saline HEPES to obtain a suspension of 7x10⁶ cells/mL. A volume of 0.8 mL of the GBK cell suspension in saline HEPES, 1 µg of BHV-1 FM gII⁺ DNA, and 0.5 µg of pΔgII digested with Pst I were added to an ice-cold 0.4 cm electroporation cuvette (BioRad). The cuvette was kept in ice for 10 min before applying 2 pulses of 280 V and 250 µF (time constant of 3-5 msec). The cuvette was left in ice for 20 sec between each pulse, and for 10 min before plating the electropored cells in a 90 mm diameter Petri dish with 15 mL of GMEM. Cells were incubated at 37°C for 5 h, then the medium removed and the cells overlayed with 15 mL of GMEM , supplemented with 2% (v/v) FCS and 0.8% (w/v) low melting agarose (BioRad) prewarmed at 42°C. Upon agarose solidification, Petri dishes were incubated at 37°C until infection areas were observed (usually, 3 days). The transformation efficiency obtained by the electroporation was approximately 2.5x10³ pfu/µg of BHV-1 FM DNA.

### Section 4.L to 4.M.: A screening method of the progeny viruses, obtained as described in the step 4K, to identify and isolate IBRV virus particles which have mutated in the locus corresponding to gII glycoprotein gene.

### 4.L. Detection of the BHV-1 FM gII⁻ virus.

When plaques on the cell monolayer were seen, 10 mL of 1.8% (w/v) DNA-grade agarose (Ecogen) were added onto the solid agarose-medium in the Petri dishes and kept at room temperature until solidification. This agarose layer confers consistency to the low melting agarose-medium. Then, the Petri dish and the agarose layers were labeled and both layers were carefully detached from the Petri dish and kept at 4°C. Next, dishes containing cell monolayers were rinsed twice with 10 mL of a buffer containing 0.8% (w/v) sodium chloride, 0.144% (w/v) sodium hydrogenphosphate, 0.024% (w/v) potassium hydrogenphosphate, and 0.02% (w/v) potassium chloride (hereinafter, "PBS"). Then, the cell monolayer was dehydrated with 10 mL of absolute ethanol for 10 min, and rehydrated with successive washes (10 min per wash) of 70% (v/v) ethanol, 50% (v/v) ethanol, and 30% (v/v) ethanol. Then, the cell monolayer was rinsed twice with PBS containing 5 mM MgCl₂ and treated 20 min with 10 mL of 0.2 M HCl. The dishes were then incubated for 30 min at 50°C in 2xSSC, 5 mM EDTA. Next, the cell monolayer was soaked for 15 min at 37°C in 5 mL of PBS, containing 1 µg/mL of proteinase K (Boehringer Mannheim) . After protease treatment, the cell monolayer was washed twice for 5 min with 10 mL of PBS containing 0.2% (w/v) glycine, and fixed for 30 min with 10 mL of PBS containing 4% (w/v) paraformaldehyde. Cell monolayers were washed twice for 10 min with 10 mL of PBS containing 5 mM MgCl₂, and then 10 mL of prehybridization solution were added. Dishes were heated at 95°C for 3 min, and incubated at 42°C for 20 min. Dishes were heated again 3 min at 95°C and ice-cooled. The prehybridization solution was removed and 3 mL of hybridization solution containing the DIG-labeled DNA probe were added onto the cell monolayer. In the present invention, the DNA probe was the 570 bp Sal I-Sma I fragment obtained during the subcloning for sequencing and named Sal A3 fragment (section **4.G.**).

The probe was obtained as follows: 5 µg of hybrid plasmid DNA from the subclone Sal A3 were incubated at 37°C for 3 h with 2 units of Sal I in 20 µL of a buffer containing 50 mM Tris-HCl (pH 7.5), 10 mM MgCI₂, 100 mM sodium chloride, and 1 mM dithiothreitol. Then, 1/10 volumes of 3 M sodium acetate were added to the above solution, which was extracted with an equal volume of phenol/chloroform/isoamylalcohol (25/24/1 v/v/v) and DNA precipitated with 2 volumes of ethanol. The pellet was redissolved in 20 µL of TE buffer and incubated at 25°C for 3 h with 2 units of Sma I in 20 µL of a buffer containing 33 mM Tris-acetate (pH 7.9), 10 mM MgCl₂, 66 mM potassium acetate, and 0.5 mM dithiothreitol. DNA was electrophoresed in a 1% (w/v) agarose gel and the 570 bp fragment was isolated and purified by Geneclean. This fragment (hereinafter "gII⁻ probe") was finally labeled with DIG as described in section **4.F.**

After detection according to the procedures described in section **4.E.**, plaques derived from the progeny of non-recombinant BHV-1 FM viruses (hereinafter "hybridized plaques") were identified by the presence of a dark precipitate (Figure 7B). Plaques not surrounded by a dark precipitate (Figure 7B) were derived from the progeny of mutant BHV-1 FM gII⁻ (hereinafter "non-hybridized plaques"). We obtained a frequency of 2.2 recombinant (mutant) viruses per 100 pfu.

### 4.M. Purification of the BHV-1 FM gII⁻ progeny.

Non-hybridized plaques were marked and rescued from agarose layer (kept at 4°C) . Pieces of about 0.5x0.5 cm were cut from the agarose corresponding to those non-hybridized plaques. Four agarose pieces (named G1, E7, E8, and E9) were placed in microfuge tubes containing 0.5 mL of GMEM, and were gently shaked for 12 h at 4°C. Viral suspensions from each non-hybridized plaque were titulated (see section **4.A.**), yielding an average of 60 pfu/µL. According to the procedure described in section **4.A.**, a volume of 1 µL from each viral suspension was used to infect the corresponding GBK cell monolayer on a Petri dish. The infected monolayers were incubated for 72 h to allow the apparition of plaques with cytopathic effect. One infected monolayer (E7) was used to perform the gII⁻ probe hybridization analysis above described. The result of the hybridization showed 8 recombinant viruses per 100 pfu, indicating a 3.5-fold enrichment. A total of 96 plaques from two of the remaining dishes (G1 and E9), were selected and numbered. Each plaque was picked with a sterile toothpick and soaked in 200 µL of GMEM into a 96-wells dish. After picking, the agarose layers were detached from the Petri dishes and the cell monolayer was hybridized with the gII⁻ probe according the above described procedure. Four clones (named: G1B-1, G1B-9, E9A-47, and E9B-22) of mutant BHV-1 FM gII⁻ viruses were isolated. Finally, confluent GBK cell monolayers growing in Petri dishes were infected (as described in section **4.A.**) with 20 µL of the viral suspensions corresponding to each one of the four clones. After 72 h, the infected monolayers were hybridized with the gII⁻ probe, according to the procedure described in section **4.L.** All plaques were not-hybridized, indicating the homogeneity of the viral progeny. The scaled-up virus production and purification was done as described in section **4.B.**.

### Section 4.N.: Analyses of purified BHV-1 gII⁻ DNA to check that progeny viruses isolated in the step 4.M. are homogeneous and have a deletion in the gII locus.

Mutant viral DNA was prepared from the recombinant clone E9B-22 according to the procedure described in section **4.B.**. The obtained DNA was digested with Hind III and the restriction fragments were electrophoresed in a 0.8% (w/v) agarose gel in TAE buffer at 16 V for 16 h at room temperature. Hind Illdigested DNA from the parental (wild-type) BHV-1 FM virus was included as a control.

The gel was stained with 0.5 µg/mL ethidium bromide in TAE buffer and photographed on an UV-transilluminator. Results are shown in Figure 7A. The restriction pattern from E9B-22 shows the lack of the 8.4 kb Hind III K, 15.9 kb Hind III C and 12.8 kb Hind III F bands. This agrees with the removal of the Hind III site in position 1504 (see Table 1) located within the gII gene. At the same time, two new bands are generated: (1) a 24.7 kb band corresponding to the fusion of the Hind III K and Hind III C fragments in one of the BHV-1 FM isoforms, and (2) a 21.2 kb band, corresponding to the fusion of the Hind IIIK and Hind IIIF fragments of the second BHV-1 FM isoform. The last band comigrates with the Hind III A band.

The above result, together with the fact that plaques derived from E9B-22 clone do not hybridize with the gII⁻ probe, shows in a conclusive manner that the recombinant virus from E9B-22 clone lacks a 1.6 kb fragment from the region coding for the BHV-1 FM gII gene. Moreover a mutant virus has been produced by homologous recombination between the parental BHV-1 FM viral strain and the pΔgII plasmid.

The clone E9B-22 has been selected for further studies and named BHV-1 FM gII⁻ and has been deposited in the Collection Nationale de Cultures de Microorganismes from Institut Pasteur, n^{º} I-1393.

### 4.O Sequences list.

SEQ ID NO:1

LOCUS          BHV-1(gII)     2336 bp double strand lineal genomic DNA

DEFINITION  gII glycoprotein gene from BHV-1 FM

CHARACTERISTICS               Localization/Features

               misc           165..170

                              /note="First GC box of gII glycoprotein"

               misc           221..226

                              /note="Second GC box of gII glycoprotein"

misc.          259..264
               /note="TATA box GC box of gII glycoprotein"
misc           288..294
               /note="CAP box of gII glycoprotein"
CDS            391..2117
               /standard_name="gII"
               /product="gII glycoprotein"
signal_pept..  391..467
               /note="precursor of gII glycoprotein"
               /start codon=1
ORIGEN OF THE MOLECULE: (a) Organism:Bovine Herpesvirus type1
                        (b) Strain:BHV-1 FM
BASE COMPOSITION        345 A    865 C    780 G    346 T
CGCGCGGGGC GGCCCCCCTG CCGCGGCGCG GCACCGAGCG CCCCAGCCAC CGTCGTGGCA   60
CTGGGCTCCC GGCCAAAGGC GCCCCCCCTC GCCACCATCA GCGAAGAATA AACGCCGCGC  120
GCGGCAAACG ATCTCGCTCG CGTGTGTCTT GGTTTCTGCG CGGCGGGCGG GGTGGGGAGC  180
GGGCAAAGCG GAGGAAGACC GGGGGCAGGA GCTGCGTGGA GGGCGGAGCC GTTGAGCGGC  240
CCGACCGCCG CCGGGTTGTT AAATGGGTCT CGCACGGCTC GTGGTTCCAC ACCGCGCCGG  300
AGAACCAGCG CGAGCTTCGC TGCGTGTGTC CCGCGAGCTG CGTTCCGGGG AACGGCGCAC  360
GCGAGAGGGT TCGAAAAGGG CATTTGGCA ATG CAA CCC ACC GCG CCG CCC CGG   413
                                Met Gln Pro Thr Ala Pro Pro Arg
                                1               5
CGG CGG TTG CTG CCG CTG CTG CTG CCG CAG TTA TTG CTT TTC GGG CTG   461
Arg Arg Leu Leu Pro Leu Leu Leu Pro Gln Leu Leu Leu Phe Gly Leu
    10                  15                  20
ATG GCC GAG GCC AAG CCC GCG ACC GAA ACC CCG GGC TCG GCT TCG GTC   509
Met Ala Glu Ala Lys Pro Ala Thr Glu Thr Pro Gly Ser Ala Ser Val
25                  30                  35                  40

GAC ACG GTC TTC ACG GCG CGC GCT GGC GCG CCC GTC TTT CTC CCA GGG 557
Asp Thr Val Phe Thr Ala Arg Ala Gly Ala Pro Val Phe Leu Pro Gly
                45                  50                  55
CCC GCG GCG CGC CCG GAC GTG CGC GCC GTT CGC GGC TGG AGC GTC CTC 605
Pro Ala Ala Arg Pro Asp Val Arg Ala Val Arg Gly Trp Ser Val Leu
            60                  65                  70
GCG GGC GCC TGC TCG CCG CCC GTG CCG GAG CCC GTC TGC CTC GAC GAC 653
Ala Gly Ala Cys Ser Pro Pro Val Pro Glu Pro Val Cys Leu Asp Asp
        75                  80                  85
CGC GAG TGC TTC ACC GAC GTG GCC CTG GAC GCG GCC TGC CTG CGA ACC 701
Arg Glu Cys Phe Thr Asp Val Ala Leu Asp Ala Ala Cys Leu Arg Thr
    90                  95                  100
GCC CGC GTG GCC CCG CTG GCC ATC GCG GAG CTC GCC GAG CGG CCC GAC 749
Ala Arg Val Ala Pro Leu Ala Ile Ala Glu Leu Ala Glu Arg Pro Asp
105                 110                 115                 120
TCA ACG GGC GAC AAA GAG TTT GTT CTC GCC GAC CCG CAC GTC TCG GCG 797
Ser Thr Gly Asp Lys Glu Phe Val Leu Ala Asp Pro His Val Ser Ala
                125                 130                 135
CAG CTG GGT CGC AAC GCG ACC GGG GTG CTG ATC GCG GCC GCA GCC GAG 845
Gln Leu Gly Arg Asn Ala Thr Gly Val Leu Ile Ala Ala Ala Ala Glu
            140                 145                 150
GAG GAC GGC GGC GTG TAC TTC CTG TAC GAC CGG CTC ATC GGC GAC GCC 893
Glu Asp Gly Gly Val Tyr Phe Leu Tyr Asp Arg Leu Ile Gly Asp Ala
        155                 160                 165
GGC GAC GAG GAG ACG CAG TTG GCG CTG ACG CTG CAG GTC GCG ACG GCC 941
Gly Asp Glu Glu Thr Gln Leu Ala Leu Thr Leu Gln Val Ala Thr Ala
    170                 175                 180
GGC GCG CAG GGC GCC GCG CGG GAC GAG GAG AGG GAA CCA GCG ACC GGG 989

Gly Ala Gln Gly Ala Ala Arg Asp Glu Glu Arg Glu Pro Ala Thr Gly
185                 190                 195                 200
CCC ACC CCC GGC CCG CCG CCC CAC CGC ACG ACG ACA CGC GCG CCC CCG 1037
Pro Thr Pro Gly Pro Pro Pro His Arg Thr Thr Thr Arg Ala Pro Pro
                205                 210                 215
CGG CGG CAC GGC GCG CGC TTC CGC GTG CTG CCG TAC CAC TCC CAC GTA 1085
Arg Arg His Gly Ala Arg Phe Arg Val Leu Pro Tyr His Ser His Val
            220                 225                 230
TAC ACC CCG GGC GAT TCC TTT CTG CTA TCG GTG CGT CTG CAG TCT GAG 1133
Tyr Thr Pro Gly Asp Ser Phe Leu Leu Ser Val Arg Leu Gln Ser Glu
        235                 240                 245
TTT TTC GAC GAG GCT CCC TTC TCG GCC AGC ATC GAC TGG TAC TTC CTG 1181
Phe Phe Asp Glu Ala Pro Phe Ser Ala Ser Ile Asp Trp Tyr Phe Leu
    250                 255                 260
CGG ACG GCC GGC GAC TGC GCG CTC ATC CGC ATA TAC GAG ACG TGC ATC 1229
Arg Thr Ala Gly Asp Cys Ala Leu Ile Arg Ile Tyr Glu Thr Cys Ile
265                 270                 275                 280
TTC CAC CCC GAG GCA CCG GCC TGC CTG CAC CCC GCC GAC GCG CAG TGC 1277
Phe His Pro Glu Ala Pro Ala Cys Leu His Pro Ala Asp Ala Gln Cys
                285                 290                 295
AGC TTC GCG TCG CCG TAC CGC TCC GAG ACC GTG TAC AGC CGG CTG TAC 1325
Ser Phe Ala Ser Pro Tyr Arg Ser Glu Thr Val Tyr Ser Arg Leu Tyr
            300                 305                 310
GAG CAG TGC CGC CCG GAC CCT GCC GGT CGC TGG CCG CAC GAG TGC GAG 1373
Glu Gln Cys Arg Pro Asp Pro Ala Gly Arg Trp Pro His Glu Cys Glu
        315                 320                 325
GGC GCC GCG TAC GCG GCG CCC GTT GCG CAC CTG CGT CCC GCC AAT AAC 1421
Gly Ala Ala Tyr Ala Ala Pro Val Ala His Leu Arg Pro Ala Asn Asn

    330                 335                 340

AGC GTA GAC CTG GTC TTT GAC GAC GCG CCG GCT GCG GCC TCC GGG CTT 1469

Ser Val Asp Leu Val Phe Asp Asp Ala Pro Ala Ala Ala Ser Gly Leu

345                 350                 355                 360

TAC GTC TTT GTG CTG CAG TAC AAC GGC CAC GTG GAA GCT TGG GAC TAC 1517

Tyr Val Phe Val Leu Gln Tyr Asn Gly His Val Glu Ala Trp Asp Tyr

                365                 370                 375

AGC CTA GTC GTT ACT TCG GAC CGT TTG GTG CGC GCG GTC ACC GAC CAC 1565

Ser Leu Val Val Thr Ser Asp Arg Leu Val Arg Ala Val Thr Asp His

            380                 385                 390

ACG CGC CCC GAG GCC GCA GCC GCC GAC GCT CCC GAG CCA GGC CCA CCG 1613

Thr Arg Pro Glu Ala Ala Ala Ala Asp Ala Pro Glu Pro Gly Pro Pro

        395                 400                 405

CTC ACC AGC GAG CCG GCG GGC GCG CCC ACC GGG CCC GCG CCC TGG CTT 1661

Leu Thr Ser Glu Pro Ala Gly Ala Pro Thr Gly Pro Ala Pro Trp Leu

    410                 415                 420

GTG GTG CTG GTG GGC GCG CTT GGA CTC GCG GGA CTG GTG GGC ATC GCA 1709

Val Val Leu Val Gly Ala Leu Gly Leu Ala Gly Leu Val Gly Ile Ala

425                 430                 435                 440

GCC CTC GCC GTT CGG GTG TGC GCG CGC CGC GCA AGC CAG AAG CGC ACC 1757

Ala Leu Ala Val Arg Val Cys Ala Arg Arg Ala Ser Gln Lys Arg Thr

                445                 450                 455

TAC GAC ATC CTC AAC CCC TTC GGG CCC GTA TAC ACC AGC TTG CCG ACC 1805

Tyr Asp Ile Leu Asn Pro Phe Gly Pro Val Tyr Thr Ser Leu Pro Thr

            460                 465                 470

AAC GAG CCG CTC GAC GTG GTG GTG CCA GTT AGC GAC GAC GAA TTT TCC 1853

Asn Glu Pro Leu Asp Val Val Val Pro Val Ser Asp Asp Glu Phe Ser

        475                 480                 485

CTC GAC GAA GAC TCT TTT GTG GAT GAC GAC AGC GAC GAT GAC GGG CCC 1901
Leu Asp Glu Asp Ser Phe Val Asp Asp Asp Ser Asp Asp Asp Gly Pro
    490                 495                 500
GCT AGC AAC CCC CCT GCG GAT GCC TAC GAC CTC GCC GGC GCC CCA GAG 1949
Ala Ser Asn Pro Pro Ala Asp Ala Tyr Asp Leu Ala Gly Ala Pro Glu
505                 510                 515                 520
CCA ACT AGC GGG TTT GCG CGA GCC CCC GCC AAC GGC ACG CGC TCG AGT 1997
Pro Thr Ser Gly Phe Ala Arg Ala Pro Ala Asn Gly Thr Arg Ser Ser
                525                 530                 535
CGC TCT GGG TTC AAA GTT TGG TTT AGG GAC CCG CTT GAA GAC GAT GCC 2045
Arg Ser Gly Phe Lys Val Trp Phe Arg Asp Pro Leu Glu Asp Asp Ala
            540                 545                 550
GCG CCA GCG CGG ACC CCG GCC GCA CCA GAT TAC ACC GTG GTA GCA GCG 2093
Ala Pro Ala Arg Thr Pro Ala Ala Pro Asp Tyr Thr Val Val Ala Ala
        555                 560                 565
CGA CTC AAG TCC ATC CTC CGC TAG GCGCCCCCCC CCCGCGCGCT GTGCCGTCTG 2147
Arg Leu Lys Ser Ile Leu Arg End
    570                 575
ACGGAAAGCA CCCGCGTGTA GGGCTGCATA TAAATGGAGC GCTCACACAA AGCCTCGTGC 2207
GGCTGCTTCG AAGGCATGGA GAGTCCACGC AGCGTCGTCA ACGAAAACTA TCGAGGCGCT 2267
GATGAGGCCG ATGCAGCGCC CCCTTCACCG CCGCCGGAAG GCTCCATCGT GTCCATCCCC 2327
ATCCTCGAG 2336

## Claims

**Claim 1.** An infectious bovine rhinotracheitis virus which fails to produce any antigenic glycoprotein gII polypeptide as a result of a deletion, an insertion or a substitution (deletion and insertion) in the bovine herpesvirus 1 (BHV-1) gII gene and/or in any control sequence of this gene, and whose characteristics identify it as BHV-1 FM gII⁻ (Collection Nationale de Cultures de Microorganismes, of the Institut Pasteur No. I-1393).

**Claim 2.** A mutant virus as claimed in Claim 1, wherein said BHV-1 FM gII⁻ virus fails to produce any antigenic glycoprotein gII polypeptide as a result of a deletion, of variable size but always compatible with the virus viability, in the BHV-1 gII gene and/or in any control sequence of this gene.

**Claim 3.** A mutant virus as claimed in Claim 2, wherein said deletion is about 1 to 1619 bp in size.

**Claim 4.** A mutant virus as claimed in Claim 1,wherein said virus fails to produce any antigenic glycoprotein gII polypeptide as a result of an insertion, of variable size but always compatible with the virus viability, in the BHV-1 gII gene and/or in any control sequence of this gene.

**Claim 5.** A mutant virus as claimed in Claim 4, wherein said insertion is about 1 to 1400 bp in size.

**Claim 6.** A mutant virus as claimed in Claim 1, wherein said virus fails to produce any antigenic glycoprotein gII polypeptide as a result of a substitution (deletion and insertion) of variable size but always compatible with the virus viability, in the BHV-1 gII gene and/or in any control sequence of this gene.

**Claim 7.** A mutant virus as claimed in Claim 6, wherein said substitution is about 1 to 3072 bp in size.

**Claim 8.** The infectious bovine rhinotracheitis virus as claimed in Claim 1, wherein said virus is Iyophilized.

**Claim 9.** A mutant virus as claimed in Claim 1, wherein said virus fails to produce some of the antigenic determinants of glycoprotein gII as a result of a deletion, an insertion or a substitution (deletion and insertion) in the BHV-1 gII gene.

**Claim 10.** A procedure for the obtention of a mutant Bovine Infectious Rhinotracheitis virus which fails to produce any antigenic glycoprotein gII polypeptide as a result of a deletion in the DNA of the BHV-1 gII gene, comprising the following steps:
(1) Constructing a first hybrid plasmid comprising the DNA of a cloning vector and a DNA fragment of the BHV-1 which includes 1200 bp of the 5' flanking region of the BHV-1 glycoprotein gII gene and 104 nucleotides of the coding region of the same gene;
(2) Constructing a second hybrid plasmid comprising the DNA of a cloning vector and a DNA fragment which includes 1740 bp of the 3' flanking region of the BHV-1 glycoprotein gII gene and the first nucleotide of the stop codon of the same gene;
(3) Constructing a third hybrid plasmid comprising the DNA of a cloning vector and the DNA fragment of the BHV-1 described in the step (1) ligated to the DNA fragment of the BHV-1 described in the step (2). This third hybrid vector carries a deletion of 1619 bp in the glycoprotein gII gene;
(4) Co-transfecting, in BHV-1 host cells, the hybrid plasmid described in step (3) with BHV-1 DNA;
and
(5) Screening the progeny viruses obtained in step (4) so as to identify and produce BHV-1 mutants which fail to produce any antigenic glycoprotein gII polypeptides as a result of a deletion in the glycoprotein gII gene.

**Claim 11.** A procedure as claimed in Claim 10, wherein the resulting hybrid plasmid of step (3) is pΔgII.

**Claim 12.** A procedure as claimed in Claim 10, wherein the resulting virus is lyophilized.

**Claim 13.** A procedure as claimed in Claim 10, wherein a foreign sequence is inserted in the place of the deleted BHV-1 glycoprotein gII gene in step (3) such that the BHV-1 DNA sequences adjacent to each side of the deleted BHV-1 gII sequence are retained and such that the resulting BHV-1 mutants of step (4) fail to produce any antigenic BHV-1 glycoprotein gII polypeptide as a result of an insertion in the BHV-1 glycoprotein gll gene.

**Claim 14.** A procedure as claimed in Claim 13, wherein the foreign DNA sequence is about 1 to 1400 bp in size.

**Claim 15.** A procedure as claimed in Claim 10, wherein all or part of the DNA sequence of glycoprotein gII gene is substituted by a foreign DNA sequence, such that the resulting BHV-1 mutants of step (4) fail to produce any antigenic BHV-1 glycoprotein gII polypeptide as a result of a substitution in the DNA corresponding to the BHV-1 glycoprotein gII gene.

**Claim 16.** A procedure as claimed in Claim 15, wherein the foreign DNA sequence is about 1 to 3072 bp in size.

**Claim 17.** A vaccine for Infectious Bovine Rhinotracheitis comprising:
(1) A pharmaceutically effective amount of BHV-1 FM gII⁻ virus, as claimed in Claim 1, which fails to produce any antigenic glycoprotein gII polypeptide as a result of a deletion, an insertion or a substitution (deletion and insertion) in the DNA corresponding to the BHV-1 glycoprotein gII gene;
and
(2) A pharmaceutically acceptable carrier or diluent.

**Claim 18**. A vaccine as claimed in Claim 17, wherein said BHV-1 FM gII⁻ virus, fails to produce any antigenic glycoprotein gII polypeptide as a result of a deletion, of variable size but always compatible with the virus viability, in the BHV-1 gII gene and/or in any control sequence of this gene.

**Claim 19.** A vaccine as claimed in Claim 18, wherein said deletion is about 1 to 1619 bp in size.

**Claim 20:** A vaccine as claimed in Claim 17, wherein said BHV-1 FM gII⁻ virus, fails to produce any antigenic glycoprotein gII polypeptide as a result of an insertion, of variable size but always compatible with the virus viability, in the BHV-1 gII gene and/or in any control sequence of this gene.

**Claim 21.** A vaccine as claimed in Claim 20, wherein said insertion is about 1 to 1400 bp in size.

**Claim 22.** A vaccine as claimed in Claim 17, wherein said BHV-1 FM gII⁻ virus fails to produce any antigenic glycoprotein gII polypeptide as a result of a substitution (deletion and insertion), of variable size but always compatible with the virus viability, in the BHV-1 gII gene and/or in any control sequence of this gene.

**Claim 23.** A vaccine as claimed in Claim 22, wherein said substitution is about 1 to 3072 bp in size.

**Claim 24.** A vaccine as claimed in Claim 17, wherein said BHV-1 FM gII⁻ virus is lyophilized.

**Claim 25.** A vaccine as claimed in Claim 17, wherein said pharmaceutically effective amount of BHV-1 FM gII⁻ virus contained in this vaccine, is comprised between 10^{4.5} to 10⁷⁻⁰ p.f.u. for vaccine dose.

**Claim 26**. A vaccine as claimed in Claim 17, wherein the acceptable carrier or diluent is physiologically buffered medium containing 2.5 to 15% (v/v) serum free from antibodies against Infectious Bovine Rhinotracheitis.

**Claim 27**. A vaccine as claimed in Claim 26, wherein said serum is one selected from the group consisting of swine serum, horse serum, calf serum and lamb serum.

**Claim 28.** A vaccine as claimed in Claim 17, wherein said virus BHV FM gII⁻ is prepared according to the procedures described in Claim 10.

**Claim 29.** A live antiviral vaccine to control the infectious bovine rhinotracheitis comprising an effective amount of recombinant virus BHV-1 FM gII⁻ as claimed in Claim 1, and a diluent or immunological adjuvant.

**Claim 30.** A universal inactivated vaccine to control the infectious bovine rhinotracheitis, comprising an effective amount of recombinant virus BHV-1 FM gII⁻ as claimed in Claim 1, and a diluent or immunological adjuvant.

**Claim 31.** A universal vaccine as claimed in Claim 29 comprising an effective amount of recombinant virus BHV-1 FM gII⁻, mixed with either/both respiratory viruses (i.e. BVD, PI3, BRSV) and/or enteric viruses (i.e. Rotavirus, Coronavirus) both live or inactivated, and mixed or not mixed with bacteria, parts of them, toxins, or another bacterial metabolism product and immunological adjuvants to enhance the immunological response.

**Claim 32.** A universal vaccine as claimed in Claim 30 comprising an effective amount of recombinant virus BHV-1 FM gII⁻, mixed with either/both respiratory viruses (i.e. BVD, PI3, BRSV) and/or enteric viruses (i.e. Rotavirus, Coronavirus) both live or inactivated, and mixed or not with bacteria, parts of them, toxins or other metabolism products and immunological adjuvants to enhance the immunological response.

**Claim 33**. A method to obtain a live vaccine as claimed in Claim 29, characterized by:
a) The replication of BHV-1 FM gII⁻ viruses in any permissive cell line (i.e., MDBK, GBK, or primary cultures) at temperatures between 36°C and 38°C.
b) The virus harvesting when the cell monolayer exhibits the highest cytopathic effect (usually between 40-50 hours post-infection).
c) The lyophilization of the live virus suspension previously mixed with stabilizing agents and/or immunological adjuvants or when the Iyophilized virus and diluents or immunological adjuvants are in separate vials which are mixed just before the administration.

**Claim 34.** A method to obtain an inactivated vaccine as claimed in Claim 30 comprising:
a) The replication of BHV-1 FM gII⁻ viruses in any permissive cell line (i.e., MDBK, GBK, or primary cultures) at temperatures between 36°C and 38°C.
b) The virus harvesting when the cell monolayer exhibits the highest cytopathic effect (usually between 40-50 hours post-infection).
c) The inactivation of the harvested virus by adding inactivating agents (i.e. formaldehyde, aziridins, BPL) at appropriate temperature and time.
d) The emulsification of the inactivated harvested virus obtained in c) by using either oily adjuvants, formulated either o/w, w/o, or w/o/w (preferentially Markol® or Drakeol®), or non-oily adjuvants (i.e. saponines, aluminium hydroxide, etc.).

**Claim 35.** A method as claimed in Claim 29 wherein said lyophilized virus is dissolved into an inactivated vaccine containing other viruses and/or bacteria in any combination.

**Claim 36.** A method as claimed in Claim 30 wherein said inactivated viruses are mixed with other viruses inactivated or not, mixed with immunological adjuvants or not, in order to obtain a vaccine with a broader antigenic spectrum.
